(19) Europäisches Patentamt
European Patent Office
Office européen des brevets

(11) Veröffentlichungsnummer: **0 183 190**
**B1**

# (12) EUROPÄISCHE PATENTSCHRIFT

(45) Veröffentlichungstag der Patentschrift: **28.12.88**

(51) Int. Cl.[4]: **C 07 C 121/75**, C 07 C 120/00, A 61 K 31/275

(21) Anmeldenummer: **85114788.4**

(22) Anmeldetag: **21.11.85**

(54) **Benzamide und Salze.**

(30) Priorität: **23.11.84 CH 5608/84**
**04.06.85 CH 2363/85**

(43) Veröffentlichungstag der Anmeldung: **04.06.86 Patentblatt 86/23**

(45) Bekanntmachung des Hinweises auf die Patenterteilung: **28.12.88 Patentblatt 88/52**

(84) Benannte Vertragsstaaten: **AT BE CH DE FR GB IT LI LU NL SE**

(56) Entgegenhaltungen:
**EP-A- 0 124 476**
**US-A- 3 219 528**

(73) Patentinhaber: **CIBA-GEIGY AG, Klybeckstrasse 141, CH-4002 Basel (CH)**

(72) Erfinder: **Storni, Angelo, Dr., Im Feuerbusch 3, CH-4310 Rheinfelden (CH)**
Erfinder: **Bischoff, Serge François, Dr., Rue Franken 10, F-68130 Altkirch-Zaessingue (FR)**
Erfinder: **von Sprecher, Georg, Dr., Bettenstrasse 64, CH-4123 Allschwil (CH)**

(74) Vertreter: **Zumstein, Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 183 190 B1

## Beschreibung

Die Erfindung betrifft Benzamide der Formel

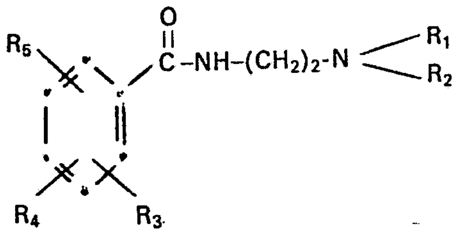

(I)

worin $R_1$ und $R_2$ unabhängig voneinander Niederalkyl bedeuten, $R_3$ Niederalkoxy, $C_3$–$C_5$-Alkenyloxy, $C_3$–$C_7$-Cycloalkoxy oder $C_3$–$C_7$-Cycloalkyl-niederalkoxy bedeutet, $R_4$ Halogen bedeutet und $R_5$ Cyano ist, und deren Salze, Verfahren zu deren Herstellung, diese Verbindungen oder ein pharmazeutisch verwendbares Salz davon enthaltende pharmazeutische Präparate und die Verwendung dieser Verbindungen, z.B. in einem Verfahren zur therapeutischen Behandlung des menschlichen oder tierischen Körpers oder zur Herstellung pharmazeutischer Präparate.

Salze der Verbindungen der Formel I sind deren Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Citronensäure, oder mit Sulfonsäuren, wie Niederalkan- oder gegebenenfalls substituierten Benzolsulfonsäuren, z.B. Methan- oder p-Toluolsulfonsäure, gebildet. Umfasst sind ferner für pharmazeutische Verwendungen ungeeignete Salze, da diese beispielsweise für die Isolierung bzw. Reinigung freier erfindungsgemässer Verbindungen sowie derer pharmazeutisch verwendbarer Salze verwendet werden können.

Vor- und nachstehend sind unter mit «nieder» bezeichneten Resten oder Verbindungen insbesondere solche zu verstehen, die bis und mit 7, vor allem bis und mit 4 Kohlenstoffatomen enthalten.

Niederalkyl ist z.B. Methyl, Ethyl, n-Propyl, Isopropyl, n-Butyl, Isobutyl, sek.-Butyl oder tert.-Butyl und umfasst ferner entsprechende Pentyl-, Hexyl- und Heptylreste.

Niederalkoxy ist z.B. Methoxy, Ethoxy, n-Propyloxy, Isopropyloxy, n-Butyloxy, Isobutyloxy, sec.-Butyloxy oder tert.-Butyloxy.

$C_3$–$C_5$-Alkenyloxy ist z.B. Allyloxy, Methallyloxy, Crotonyloxy oder 3,3-Dimethylallyloxy.

$C_3$–$C_7$-Cycloalkoxy ist z.B. Cyclopropyloxy, Cyclobutyloxy, Cyclopentyloxy, Cycloheptyloxy.

$C_3$–$C_7$-Cycloalkyl-niederalkoxy ist z.B. Cyclopropyl-, Cyclobutyl-, Cyclopentyl- oder Cyclohexyl-methoxy oder -ethoxy.

Halogen ist z.B. Halogen bis und mit Atomnummer 35, wie Fluor, Chlor oder Brom, ferner Jod.

In den US-Patentschriften Nr. 3 177 252 und Nr. 3 219 528 sind antiemetisch wirksame Verbindungen mit analoger Struktur beschrieben.

Demgegenüber weisen die Verbindungen der Formel I und ihre pharmazeutisch verwendbaren Salze ein neuartiges pharmakologisches Wirkungsprofil auf.

In dem von S. Bischoff et al., European J. Pharmacology 68, 305–315 (1980), beschriebenen Testmodell wurde bei Applikation der erfindungsgemässen Verbindungen ab einer Dosis von etwa 1,0 mg/kg bei der Ratte eine Steigerung der in-vivo-[$^3$H] Spiperone-Bindung in allen relevanten Hirnstrukturen, vor allem im Striatum, festgestellt. Dieser Effekt deutet auf eine Blockade der präsynaptischen Dopamin(DA)-Rezeptoren bei niedrigen Dosen, wodurch eine Steigerung des DA-Umsatzes bewirkt wird. Die durch diese Stimulation bedingte positive Verhaltensänderung kann ebenfalls im Sozialinteraktionstest analog S. File et al., Pharmacol. Biochem. Behav. 11, 65–69 (1979), festgestellt werden. Bei kumulierter Wirkstoffkonzentration werden die postsynaptischen DA-Rezeptoren blockiert, wie, ab einer Dosis von etwa 90 mg/kg bei der Ratte, anhand der Inhibition der in-vivo[$^3$H]Spiperone-Bindung in der Hypophyse, wo lediglich postsynaptische DA-Rezeptoren lokalisiert sind, nachgewiesen werden konnte. Als Folge davon wurde im genannten Sozialinteraktionstest bei der Ratte eine Unterdrückung von motorischer Unruhe und eine Hemmung der durch Amphetamin induzierten Stereotypien beobachtet.

Besonders vorteilhaft ist die Tatsache, dass keine extrapyramidalen Nebenwirkungen bei der Anwendung der erfindungsgemässen Verbindungen festgestellt wurden.

Somit wurden erstmals Wirksubstanzen gefunden, die gleichzeitig bei niedrigen Dosen zunächst eine stimulierende und bei akkumulierter Wirkstoffkonzentration eine dämpfende Wirkung auf die Psyche bewirken.

Die Verbindungen der Formel I sowie ihre pharmazeutisch verwendbaren Salze können dementsprechend als Pharmazeutika, wie als dopaminergstimulierende Antidepressiva und als Therapeutika mit antidepressiver und neuroleptischer Komponente, insbesondere zur Behandlung von chronischer Schizophrenie und von depressiven Zuständen, verwendet werden. Bei der Verwendung der erfindungsgemässen Verbindungen ist neben der therapeutischen Anwendung auch die gewerbsmässige Herrichtung der Wirksubstanzen eingeschlossen.

Ein weiterer Gegenstand der Erfindung ist somit die therapeutische Behandlung des menschlichen oder tierischen Körpers sowie die Verwendung der erfindungsgemässen Verbindungen z.B. als Therapeutika sowie zur Herstellung pharmazeutischer Präparate.

Die Erfindung betrifft insbesondere Verbindungen der Formel I, worin $R_1$ und $R_2$ unabhängig voneinander Niederalkyl mit bis und mit 4 C-Ato-

men bedeuten, $R_3$ Niederalkoxy mit bis und mit 4 C-Atomen, $C_3$–$C_5$-Alkenyloxy, $C_3$–$C_7$-Cycloalkoxy oder $C_3$–$C_7$-Cycloalkyl-niederalkoxy mit bis und mit 4 C-Atomen im Niederalkoxyteil bedeutet, $R_4$ Halogen bis und mit Atomnummer 35 bedeutet und $R_5$ Cyano ist, und ihre Salze.

Die Erfindung betrifft insbesondere Verbindungen der Formel I, worin $R_1$ und $R_2$ unabhängig voneinander Niederalkyl mit bis und mit 4 C-Atomen bedeutet, $R_3$ Niederalkoxy mit bis und mit 4 C-Atomen bedeutet, $R_4$ Halogen bis und mit Atomnummer 35 bedeutet und $R_5$ Cyano ist, und ihre Salze.

Die Erfindung betrifft in erster Linie unter die Formel I fallende Verbindungen der Formel

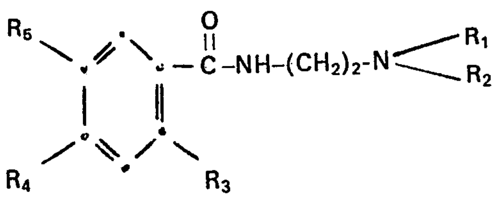

(Ia)

worin $R_1$, $R_2$, $R_3$, $R_4$ und $R_5$ die angegebenen Bedeutungen haben, und ihre Salze.

Die Erfindung betrifft in erster Linie unter die Formel I fallende Verbindungen der Formel Ia, worin $R_1$ und $R_2$ unabhängig voneinander Niederalkyl mit bis und mit 4 C-Atomen bedeutet, $R_3$ Methoxy oder Ethoxy bedeuten, $R_4$ Chlor bedeutet und $R_5$ Cyano ist, und ihre Salze.

Die Erfindung betrifft in erster Linie unter die Formel I fallende Verbindungen der Formel Ia, worin $R_1$ und $R_2$ Niederalkyl, insbesondere mit bis und mit 4 C-Atomen, bedeutet, $R_3$ Methoxy bedeutet, $R_4$ Chlor bedeutet und $R_5$ Cyano ist, und ihre Salze.

Die Erfindung betrifft in erster Linie Verbindungen der Formel I bzw. Ia, worin $R_1$ und $R_2$ unabhängig voneinander Niederalkyl bedeuten, wobei die Summe der C-Atomzahlen 4 bis und mit 6 ausmacht, und ihre Salze.

Die Erfindung betrifft vor allem Verbindungen der Formel I bzw. Ia, worin $R_1$ und $R_2$ unabhängig voneinander Ethyl oder Isopropyl bedeuten, und ihre Salze.

Die Erfindung betrifft insbesondere die in den Beispielen genannten neuen Verbindungen.

Ebenfalls Gegenstand der vorliegenden Erfindung sind Verfahren zur Herstellung der Verbindungen der Formel I und ihrer Salze, dadurch gekennzeichnet, dass man

a) in einer Verbindung der Formel

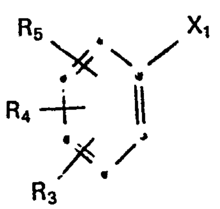

(II)

oder einem Salz davon, worin $X_1$ einen in die Gruppierung der Formel $-CO-NH-(CH_2)_2-N(R_1)(R_2)$ überführbaren Rest bedeutet, $X_1$ in die Gruppierung $-CO-NH-(CH_2)_2-N(R_1)(R_2)$ überführt, oder

b) eine Verbindung der Formel

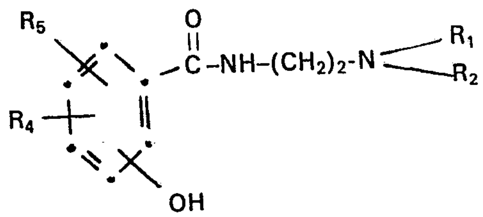

(III)

oder ein Salz davon verethert, oder

c) in einer Verbindung der Formel

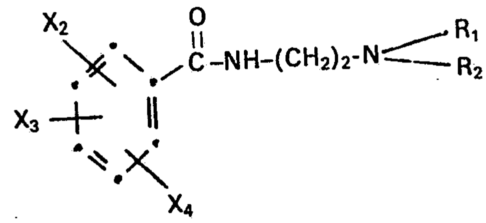

(IV)

oder einem Salz davon, worin einer der Reste $X_2$, $X_3$ und $X_4$ für die Diazoniumgruppierung $-N_2^{\oplus} A^{\ominus}$ und $A^{\ominus}$ für ein Anion steht, und die anderen Reste, entsprechend dem Substitutionsmuster der Verbindung der Formel I, $R_3$, $R_4$ bzw. $R_5$ bedeuten, die Diazoniumgruppe substituiert, oder

d) in einer Verbindung der Formel

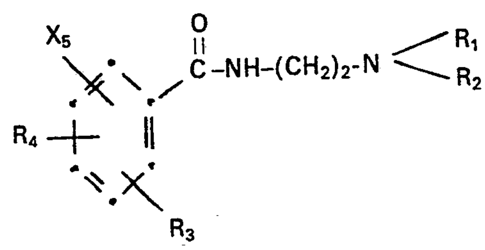

(V)

oder einem Salz davon, worin $X_5$ für einen in Cyano überführbaren Rest steht, $X_5$ in Cyano überführt, und gewünschtenfalls ein verfahrensgemäss erhältliches Salz in die freie Verbindung der Formel I oder in ein anderes Salz überführt und/oder die verfahrensgemäss erhältliche freie Verbindung in ein Salz überführt.

Ausgangsmaterial mit basischen Zentren kann z.B. in Form von Säureadditionssalzen, beispielsweise mit den vorstehend aufgeführten Säuren, vorliegen, während Ausgangsverbindungen mit sauren Gruppen, z.B. Carboxy oder phenolisches Hydroxy, Salze mit Basen, wie Alkalimetall-, Erdalkalimetall-, Ammonium- oder Aminsalze mit substituierten organischen Aminen, bilden können.

Die vor- und nachstehend in den Varianten a) bis d) beschriebenen Umsetzungen werden nach an sich bekannten Verfahren durchgeführt, beispielsweise in Ab- oder üblicherweise Anwesenheit eines geeigneten Lösungs- bzw. Verdünnungsmittels oder eines Gemisches derselben,

wobei man je nach Verfahrensweise unter Kühlen, bei Raumtemperatur oder unter Erwärmen, z.B. in einem Temperaturbereich von etwa −20° bis zur Siedetemperatur des Reaktionsmediums, und, falls erforderlich, in einem geschlossenen Gefäss, unter Druck, in einer Inertgasatmosphäre und/oder unter wasserfreien Bedingungen arbeitet.

Die vor- und nachstehend aufgeführten Ausgangsmaterialien der Formeln II, III, IV und V, die für die Herstellung der Verbindungen der Formel I und ihrer Salze entwickelt wurden, sind zum Teil bekannt bzw. können nach an sich bekannten Methoden, z.B. analog der vor- und nachstehend beschriebenen Verfahrensvarianten, hergestellt werden.

Variante a):

$X_1$ der Formel II bedeutet beispielsweise Carboxy und funktionell abgewandeltes, in erster Linie reaktionsfähiges funktionell abgewandeltes Carboxy. Zur Herstellung der Verbindungen der Formel I und ihrer Salze geht man z.B. von Carboxy- oder reaktionsfähigen funktionell abgewandelten Carboxyderivaten der Formel II aus und setzt diese mit der Verbindung der Formel $H_2N-(CH_2)_2-N(R_1)(R_2)$ (IIa) oder einem Salz davon um.

Reaktionsfähige funktionell abgewandelte Carboxyderivate der Formel II sind beispielsweise gemischte, ferner symmetrische Anhydride, aktivierte Ester und aktivierte Amide.

Als gemischte Anhydride kommen z.B. solche mit anorganischen Säuren, mit gemischten anorganischen Säureanhydriden oder mit anorganischen Estern in Frage, wie Carbonsäurehalogenide, z.B. das Chlorid, das Carbonsäureazid, gemischte Anhydride mit einem gemischten Phosphorsäureanhydrid, z.B. mit einem Phosphorylhalogenid, oder gemischte Anhydride mit einem Kohlensäurehalbester, z.B. mit einem Kohlensäureniederalkylhalbester. Gemischte Anhydride können ebenso mit organischen Säuren, wie mit unsubstituierten oder, z.B. durch Halogen, substituierten Niederalkancarbonsäuren, oder mit organischen Sulfonsäuren, wie mit gegebenenfalls, z.B. durch Halogen bzw. Niederalkyl, substituierten Niederalkan- bzw. Benzolsulfonsäuren, gebildet werden.

Unter aktivierten Estern der Carbonsäure der Formel II sind beispielsweise der Vinylester oder aktivierte Vinylester, wie 1-Niederalkoxy-vinylester oder 2-(N-Niederalkylcarbamoyl)-1-hydroxysulfonylphenyl-vinylester, Aryl(thio)ester, wie unsubstituierte oder, z.B. durch Halogen, Nitro oder Phenylhydrazo, substituierte Phenylester unsubstituierte oder, z.B. durch Nitro, substituierte Phenylthioester oder Pyridiniumester, z.B. 1-Niederalkyl-2-pyridiniumester, der Cyanmethylester, 2-Isoharnstoffester, wie 1,3-Diniederalkyl-, 1,3-Dicycloalkyl-2-isoharnstoffester oder 1-Di(phenyl)niederalkyl-2-isoharnstoffester, oder Silylester, wie Triniederalkylsilylester, zu verstehen. Ebenso sind z.B. N-Hydroxyester zu nennen, wie solche, die aus N-Hydroxy-succinimid, N-Hydroxy-phthalimid, N-Hydroxy-piperidin oder 1,1'-(Carbonyldioxy)-dibenzotriazol (führt zum 1-Benzotriazolester) gebildet werden.

Aktivierte Amide der Formel II sind beispielsweise Imidazolide, z.B. aus 1,1'-Carbonyldiimidazol gebildet, oder 3,5-disubstituierte Pyrazolide, die z.B. durch Umsetzung des Hydrazids mit einem 1,3-Diketon gebildet werden können.

Die verfahrensgemässe Umsetzung (N-Acylierung) wird erforderlichenfalls in Gegenwart eines, insbesondere basischen, Kondensationsmittels durchgeführt. Als Basen kommen beispielsweise Alkalimetallhydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide sowie carbocyclische Amine in Frage. Beispielhaft seien Natrium-hydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyl-trimethylammoniumhydroxid, 1,5-Diazabicyclo[4.3.0]non-5-en (DBN) sowie 1,8-Diazabicyclo[5.4.0]-undec-7-en (DBU) genannt. Den Kondensationsmitteln zuzurechnen sind die bei der Bildung von Amidbindungen üblichen Dehydratisierungsmittel, die insbesonders dann verwendet werden, wenn $X_1$ der Formel II Carboxy bedeutet. Dabei können beispielsweise in situ reaktionsfähige Carboxyderivate der Formel II, insbesondere entsprechende aktivierte Ester bzw. Amide, in erster Linie der vorstehend aufgeführten Art, gebildet werden. Geeignete Dehydratisierungsmittel sind beispielsweise Carbodiimide, z.B. N,N'-Diniederalkyl- oder N,N'-Dicycloalkylcarbodiimide, wie N,N'-Diethyl-, N,N'-Diisopropyl- oder N,N'-Dicyclohexyl-carbodiimide, vorteilhaft unter Zusatz von N-Hydroxysuccinimid oder gegebenenfalls, z.B. durch Halogen, Niederalkyl oder Niederalkoxy, substituiertes 1-Hydroxy-benzotriazol oder N-Hydroxy-5-norbornen-2,3-dicarboxamid, N,N'-Diimidazolcarbonyl, eine geeignete Phosphonyl-bzw. Phosphinverbindung, z.B. Diethylphosphonylcyanid, Diphenylphosphonylazid oder Triphenylphosphin-disulfid, ein 1-Niederalkyl-2-halogen-pyridinium-halogenid, z.B. 1-Methyl-2-chlor-pyridiniumiodid, ein geeignetes 1,2-Dihydrochinolin, z.B. N-Ethoxycarbonyl-2-ethoxy-1,2-dihydrochinolin, oder 1,1'-(Carbonyldioxy)-dibenzotriazol.

Reaktionsfähige funktionell abgewandelte Carboxyderivate der Formel II können in an sich bekannter Weise hergestellt werden. So kann man z.B. Carbonsäurehalogenide, insbesondere das Carbonsäurechlorid, gemischte Anhydride mit einem Phosphorylhalogenid, insbesondere das entsprechend Chlorderivat, oder gemischte Anhydride mit einem Kohlensäurehalbester erhalten, indem man die Carbonsäure der Formel II z.B. mit Thionylchlorid oder Phosphorpentachlorid; mit Phosphoroxychlorid bzw. mit einem Chlorkohlensäureniederalkylester behandelt, während zur Herstellung des Carbonsäureazids die Carbonsäure der Formel II zunächst mit Hydrazin und anschliessend mit salpetriger Säure umgesetzt wird. Der Vinylester der Formel II kann z.B. durch Umesterung eines entsprechenden Niederalkylesters z.B. mit Essigsäurevinylester, ein 1-Niederalkoxy-, insbesondere 1-Ethoxy-vinylester nach dem Ethoxyacetylen-Verfahren, ein 2-(N-Niederalkylcarbamoyl)- 1-hydroxysulfonylphenyl-vinylester analog der Woodward-Methode mit einem entsprechenden 1,2-Oxazolium-Reagens, ein Phenyl(thio)ester nach der Carbodiimid-Methode aus der Carbonsäure und dem Phenol bzw. Thiophenol, ein 1-Niederalkyl-, insbesondere 1-Methyl-2-pyridiniumester z.B. durch Reaktion mit 2-Chlor-1-methyl-pyridiniumiodid in Gegenwart einer Aminbase, ein 1,3-Diniederalkyl- bzw. 1,3-Dicycloalkyl-2-isoharnstoffester durch Umsetzung der Carbonsäure mit einem entsprechenden Diimid gemäss der Carbodiimid-Methode und ein 1-Di(phenyl)niederalkyl-2-isoharnstoffester durch Behandeln der Carbonsäure mit einem entsprechenden Cyanamid (Cyanamid-Verfahren) erhalten werden, während ein Silylester z.B. mit einem entsprechenden Chlorsilan gebildet werden kann.

Weiterhin kann man von solchen Verbindungen der Formel II ausgehen, worin $X_1$ Carbamoyl ist, und diese mit einer Verbindung der Formel $X_6$–$(CH_2)_2$–$N(R_1)(R_2)$ (IIb) oder einem Salz davon, worin $X_6$ reaktionsfähiges verestertes Hydroxy bedeutet, umsetzen.

Reaktionsfähiges verestertes Hydroxy bedeutet insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethan-sulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy.

$X_1$ der Formel II kann ebenso für N-(2-$X_6$-ethyl)-carbamoyl, Aziridin-1-yl-carbonyl oder 2-Oxazolin-2-yl stehen. Entsprechende Ausgangsverbindungen können mit einem Amin der Formel $HN(R_1)(R_2)$ (IIc) oder einem Salz davon, vorzugsweise im Überschuss, zu der Verbindung der Formel I umgesetzt werden.

Ferner kann die Behandlung von Ausgangsstoffen der Formel II, worin $X_1$ 2-(N-$X_7$-amino-ethyl)-carbamoyl bedeutet und $X_7$ Wasserstoff oder $R_1$ bzw. $R_2$ ist, mit einer Verbindung der Formel $X_6$–$R_2$ bzw. $X_6$–$R_1$ zu der Verbindung der Formel I führen.

Die vorstehend beschriebenen Umsetzungen können gegebenenfalls in Gegenwart einer der oben aufgeführten Base durchgeführt werden, wobei insbesondere in einem Temperaturintervall von etwa 20 °C bis zur Siedetemperatur des Reaktionsmediums gearbeitet wird.

Eine N-Substitution von Verbindungen der Formel II, worin $X_1$ für 2-(N-$X_7$-amino-ethyl)-carbamoyl steht, kann ebenfalls durch Umsetzung mit $R_1$ bzw. $R_2$ entsprechenden Niederalkanalen oder Niederalkanonen erfolgen. Als den Resten $R_1$ bzw. $R_2$ entsprechenden Niederalkanale bzw. Niederalkanone kommen z.B. Formaldehyd oder Acetaldehyd bzw. Aceton, ferner auch entsprechende gegebenenfalls veretherte oder veresterte Hydroxyniederalkanale bzw. -niederalkanone oder gegebenenfalls veresterte Carboxy-niederalkanale bzw. -niederalkanone in Frage.

Die Umsetzung wird üblicherweise unter reduzierenden Bedingungen durchgeführt, beispielsweise mit Wasserstoff in Gegenwart eines Hydrierungskatalysators oder mit anderen Reduktionsmitteln, insbesondere mit Ameisensäure. Hydrierungskatalysatoren sind beispielsweise Nebengruppenelemente oder deren Derivate, vorzugsweise solche der VIII. Nebengruppe, wie Palladium, Platin oder Palladium- bzw. Platindioxid, wobei die Katalysatoren auf geeigneten Trägermaterialien, wie Aktivkohle Aluminiumoxid oder Siliziumdioxid, aufgezogen sein können.

Variante b):

Die Veretherung kann beispielsweise mit Hilfe eines entsprechenden Alkylierungsmittels erfolgen. Als derartige Mittel kommen z.B. Verbindungen der Formel $R'_3$–$X_6$, wobei $R'_3$ ein von $R_3$ abgeleiteter Rest bedeutet, wie Niederalkyl, $C_3$–$C_5$-Alkenyl, $C_3$–$C_7$-Cycloalkyl oder $C_3$–$C_7$-Cycloalkylniederalkyl, und $X_6$ die vorstehend, angegebenen Bedeutungen hat. Vorzugsweise kommen Niederalkyl-, $C_3$–$C_5$-Alkenyl-, $C_3$–$C_7$-Cycloalkyl- oder $C_3$–$C_7$-Cycloalkyl-niederalkyl-halogenide, wie Methyliodid, ebenso Diniederalkylsulfate, wie Dimethylsulfat, Diazoniederalkane, wie Diazomethan, Triniederalkylsulfonium-, Triniederalkylselenium-, Triniederalkyloxosulfonium- oder Triniederalkylaniliniumhydroxide, wie Trimethylsulfonium-, Trimethylselenium-, Trimethyloxosulfonium- oder Trimethylaniliniumhydroxid, oder ein $R_3$ entsprechender Alkohol, wie Methanol, in Betracht.

Bei der Verwendung von Verbindungen der Formel $R'_3$–$X_6$ und einem Diniederalkylsulfat erfolgt die Veretherung insbesondere in Gegenwart einer der vorstehend genannten Basen, vorzugsweise von Kaliumcarbonat, während die Umsetzung mit einem Diazoniederalkan erforderlichenfalls in Gegenwart einer Lewissäure durchgeführt wird. Lewissäuren sind beispielsweise Halogenide von Bor, Aluminium, Zinn (II), Antimon (III), Arsen (III), Silber (I), Zink (II) und Eisen (III).

Die Veretherung der Verbindung der Formel III mit Hilfe eines von $R_3$ abgeleiteten Alkohols, wie Methanol wird beispielsweise in Gegenwart einer starken Säure oder, unter wasserfreien Bedingungen, eines Dehydratisierungsmittels, insbesondere der oben aufgeführten Art, durchgeführt.

Als starke Säuren seien insbesondere starke Protonsäuren, beispielsweise Mineralsäuren, wie Halogenwasserstoffsäuren, Schwefel- oder eine Phosphorsäure, starke Carbonsäuren, wie eine gegebenenfalls, z.B. durch Halogen, substituierte Niederalkancarbonsäure bzw. Benzoesäure, z.B.

Eisessig oder Trifluoressigsäure, oder Sulfonsäuren, wie gegebenenfalls, z.B. durch Halogen, substituierte Niederalkansulfonsäure oder gegebenenfalls, z.B. durch Halogen oder Niederalkyl, substituierte Benzolsulfonsäure, z.B. p-Toluolsulfonsäure, genannt.

Variante c):

Je nach Wahl des Ausgangsmaterials der Formel IV kann die Substitution der Diazoniumgruppierung $-N_2^{\oplus}$ $A^{\ominus}$ durch Behandeln mit einem Cyanid, einem Chlorid oder Methanol erfolgen.

Bedeutet $X_2$ der Formel IV die Diazonierungsgruppierung $-N_2^{\oplus}$ $A^{\ominus}$, $X_3$ $R_4$ und $X_4$ $R_3$, kann Cyano beispielsweise durch Umsetzung mit Cyaniden, z.B. analog der Sandmeyer-Reaktion mit Kupfer(I)cyanid oder Alkalimetall-tetracyanocuprat (I) oder in Anlehnung an die Gattermann-Reaktion mit Alkalimetallcyaniden in Gegenwart von metallischem Kupfer, eingeführt werden.

Die Substitution der Diazoniumgruppe $X_3$ in Verbindungen der Formel IV ($X_2$ ist $R_5$ und $X_4$ $R_3$) kann z.B. ebenfalls gemäss der Sandmeyer-Reaktion mit Kupfer(I)chlorid oder mit Chloriden, wie Alkalimetallchloriden, in Gegenwart von Kupfermetall nach Gattermann durchgeführt werden.

Behandelt man z.B. Verbindungen der Formel IV, worin $X_2$ $R_5$ ist, $X_3$ $R_4$ bedeutet und $X_4$ für die Gruppierung $-N_2^{\oplus}$ $A^{\ominus}$ steht, mit einem $R_3$ entsprechenden Alkohol, wird $X_4$ durch $R_3$ ersetzt.

In einer vorteilhaften Modifikation dieser Verfahrensvariante können die Verbindungen der Formel IV in situ gebildet werden und unter den jeweiligen Reaktionsbedingungen ohne Isolierung zu der Verbindung der Formel I weiterreagieren. Dabei geht man zunächst von entsprechenden Aminen der Formel IV aus (einer der Reste $X_2$, $X_3$ und $X_4$ ist Amino und die anderen bedeuten, entsprechend dem Substitutionsmuster der Verbindung der Formel I, $R_3$, $R_4$ bzw. $R_5$), diazotiert diese mit Nitriten, wie Alkalimetallnitriten, oder Nitroniederalkanen in Gegenwart von Protonensäuren, z.B. solchen der in Variante b) aufgeführten Art, und setzt die in situ gebildeten Verbindungen der Formel IV ohne deren Isolierung in der jeweils vorstehend beschriebenen Weise zu der Verbindung der Formel I weiter um.

Vorteilhaft wird für diese Umsetzungen eine Reaktionstemperatur von etwa −10° bis etwa +40 °C gewählt.

Variante d):

Eine Verbindung der Formel V kann beispielsweise durch Dehydratisierung in die Verbindung der Formel I oder ein Salz davon übergeführt werden. Dementsprechend steht $X_5$ der Formel V z.B. für Hydroxyiminomethyl bzw. O-substituiertes Hydroxyiminomethyl, Carbamoyl, N-monosubstituiertes Carbamoyl bzw. ein entsprechendes Thiocarbamoyl. Dabei wird substituiertes Hydroxyiminomethyl bzw. Carbamoyl in erster Linie in situ gebildet, wobei die jeweiligen Substituenten sich vorwiegend von den jeweiligen Dehydratisierungsmitteln ableiten. Derartige Reste $X_5$ sind z.B. Trihalogenniederalkanoyl-imino-methyl oder durch Halogensulfonyl, Niederalkan- oder unsubstituiertes bzw., z.B. durch Halogen oder Niederalkyl, substituiertes Benzolsulfonyl oder Aminosulfonyl substituiertes Carbamoyl.

Die Dehydratisierung erfolgt beispielsweise mit Hilfe eines für die Bildung von Nitrilen üblichen Dehydratisierungsmittels, wobei bevorzugt in Gegenwart einer der vorstehend genannten Basen gearbeitet wird. Derartige Dehydratisierungsmittel werden in Synthesis 905f. (1978) bzw. 748f. (1983) aufgeführt. Beispielhaft seien gemischte sowie symmetrische Säureanhydride genannt, die z.B. aus substituierten bzw. unsubstituierten Niederalkancarbonsäuren, Sulfonsäuren und/oder Mineralsäuren gebildet werden. Derartige Säureanhydride sind z.B. Trihalogenniederalkancarbonsäureanhydride bzw. -halogenide, Triniederalkansulfonsäureanhydride, Sulfonyl-, wie Sulfurylhalogenide, oder Phosphorhalogenide, wie Phosphor III oder V- bzw. Phosphoroxyhalogenide.

Aus der Reihe der im Zusammenhang mit Variante a) aufgeführten Dehydratisierungsmittel seien z.B. Dicyclohexylcarbodiimid, Cyanurchlorid und 1,1'-Dicarbonyl-diimidazol erwähnt.

Die Zwischenprodukte der Formel V, insbesondere solche, worin $X_5$ Hydroxyiminomethyl, O-substituiertes Hydroxyiminomethyl oder N-monosubstituiertes Carbamoyl bedeutet, werden nach an sich bekannten Verfahren zum überwiegenden Teil in situ gebildet und unter den Reaktionsbedingungen durch Isolierung weiter zu der Verbindung der Formel I umgesetzt.

So kann man in einer bevorzugten Ausführungsform der vorstehenden Verfahrensvariante z.B. von der Verbindung der Formel V ausgehen, worin $X_5$ Formyl bedeutet, und diese mit einem Hydroxylamin, insbesondere eines Säureadditionssalzes desselben, und mit einem entsprechenden Dehydratisierungsmittel behandeln. Dabei wird zunächst intermediär eine Verbindung der Formel V gebildet, worin $X_5$ Hydroxyiminomethyl bedeutet, die nicht isoliert werden braucht und unter Einwirkung des Dehydratisierungsmittels verfahrensgemäss weiterreagiert.

Die Dehydratisierung kann zunächst insbesondere bei vorgeschalteter Bildung des Aldoxims ($X_5$ = $-CH{=}N{-}OH$) aus der entsprechenden Formylverbindung ($X_5$ = $-CHO$), unter Kühlen, erforderlichenfalls bis auf −78 °C durchgeführt werden; die Reaktionstemperatur kann anschliessend auf die Siedetemperatur des Reaktionsmediums erhöht werden.

Die Bildung der Verbindung der Formel I und eines Salzes derselben kann auch durch direkte Einführung der Cyanogruppe in die Verbindung der Formel V, worin $X_5$ Wasserstoff ist, durch Reaktion mit einem Halogencyan, z.B. Chlor- oder Bromcyan, oder mit Dicyan erfolgen. Diese Umsetzung wird mit einer der oben genannten Lewissäuren als Katalysator durchgeführt.

Die Erfindung betrifft insbesondere die in den Beispielen beschriebenen Verfahren.

Weisen die genannten Ausgangsstoffe basische Zentren auf, können z.B. auch Säureadditionssalze gebildet werden, während Ausgangsstoffe mit aciden Gruppen z.B. Salze mit Basen bilden.

Je nach Wahl der Reaktionsbedingungen können die Ausgangsstoffe in freier Form oder als Salze eingesetzt werden bzw. können die erfindungsgemässen Verbindungen mit salzbildenden Eigenschaften in freier Form oder in Form von Salzen erhalten werden.

So können erhaltene Säureadditionssalze in an sich bekannter Weise, z.B. durch Behandeln mit einer Base, wie einem Alkalimetallhydroxid, in die freie Verbindung oder z.B. durch Behandeln mit geeigneten Säuren oder Derivaten davon in andere Salze umgewandelt werden. Die erhaltene freie Verbindung mit salzbildenden basischen Eigenschaften kann, z.B. durch Behandeln mit Säuren oder entsprechenden Anionenaustauschern, in ihre Salze umgewandelt werden.

Infolge der engen Beziehung zwischen den salzbildenden Verbindungen in freier Form und in Form von Salzen, sind im Vorausgegangenen und nachfolgend unter den freien Verbindungen oder ihren Salzen sinn- und zweckmässig gegebenenfalls auch die entsprechenden Salze bzw. freien Verbindungen zu verstehen. Umfasst sind ferner für pharmazeutische Verwendungen ungeeignete Salze, da diese beispielsweise für die Isolierung bzw. Reinigung freier erfindungsgemässer Verbindungen sowie deren pharmazeutisch verwendbarer Salze verwendet werden können.

Die erfindungsgemässen Verbindungen, einschliesslich ihrer Salze, können auch in Form ihrer Hydrate erhalten werden, oder ihre Kristalle können z.B. das zur Kristallisation verwendete Lösungsmittel einschliessen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, ihre Verwendung, z.B. als Zwischenprodukte, ferner gegebenenfalls als Arzneimittelwirkstoffe, und Verfahren zu ihrer Herstellung, bilden ebenfalls einen Gegenstand der Erfindung.

Die Dosierung des Wirkstoffes, der allein oder zusammen mit dem üblichen Träger- und Hilfsmaterial verabreicht wird, hängt von den zu behandelnden Spezies, deren Alter und individuellen Zustand sowie der Verabreichungsweise ab. Die Einzeldosen liegen z.B. für Mammalien mit einem Körpergewicht von ca. 75 kg, je nach Art der Erkrankung, individuellem Zustand und Alter, vorzugsweise bei etwa 100 mg, z.B. bei oraler Verabreichung.

Die Erfindung betrifft im weiteren pharmazeutische Präparate und Verfahren zur Herstellung pharmazeutischer Präparate, die Verbindungen der Formel (I) oder pharmazeutisch verwendbare Salze von solchen Verbindungen mit salzbildenden Eigenschaften als Wirkstoffe enthalten.

Bei den erfindungsgemässen pharmazeutischen Präparaten handelt es sich um solche zur enteralen, wie peroralen oder rektalen, weiter zur parenteralen Verabreichung an Warmblüter. Entsprechende Dosiseinheitsformen, insbesondere zur peroralen Verabreichung, z.B. Dragées, Tabletten oder Kapseln, enthalten vorzugsweise von etwa 1 mg bis etwa 100 mg, insbesondere von etwa 1 mg bis etwa 25 mg, einer Verbindung der Formel (I) oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaftresistenten, Überzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, oder Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können. Bevorzugt sind u.a. Kapseln, die sowohl leicht zerbissen als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1:

Zu einer Lösung von 11,6 g (0,1 Mol) 2-Diethylaminoethylamin in 100 ml Methylenchlorid wird unter Stickstoff und Rühren bei minus 30° eine Lösung von 23 g (0,1 Mol) 4-Chlor-5-cyano-2-methoxy-benzoesäurechlorid in 70 ml Methylenchlorid getropft. Anschliessend lässt man die entstandene weisse Suspension 15 Stunden bei Raumtemperatur rühren, tropft dann 105 ml (0,105 Mol) 1 M Natronlauge zu, rührt bis 2 klare Schichten entstanden sind, trennt im Scheidetrichter und schüttelt die wässrige Phase nochmals mit 50 ml Methylenchlorid aus. Die vereinigten organischen Phasen werden über Magnesiumsulfat getrocknet, filtriert, mit 150 ml Cyclohexan versetzt und Methylenchlorid abdestilliert bis die Destillationstemperatur 70° beträgt. Nach dem Abkühlen wird von der auskristallisierten Base genutscht und mit Cyclohexan nachgewaschen. Nach dem Trocknen wird 4-Chlor-5-cyano-N- (2-diethylamino-ethyl)- 2-methoxybenzamid erhalten mit einem Schmelzpunkt von 104–105°.

Beispiel 2:

30 g 4-Chlor-5-cyano-N-(2-diethylaminoethyl)-2-methoxybenzamid werden in 100 ml Aceton gelöst und unter Rühren und Eiskühlung mit etherischer Chlorwasserstoffsäure auf pH 5 gestellt, wobei das Hydrochlorid auskristallisiert. Man nutscht, wäscht 2× mit wenig Aceton nach, trocknet das Salz am Hochvakuum bei 60° und erhält 4-Chlor- 5-cyano-N- (2-diethylaminoethyl)-2-methoxy-benzamid-hydrochlorid mit einem Schmelzpunkt von 189–190° (Zers.).

Beispiel 3:

25,5 g (0,1 Mol) N-(2-Amino-ethyl)-4-chlor-5-cyano-2-methoxy-benzamid, 39 g (0,25 Mol) Ethyljodid und 41,5 g Kaliumcarbonat werden in 300 ml Ethanol während 15 Stunden bei 50° gerührt. Anschliessend wird das Ethanol im Wasserstrahlvakuum eingedampft und der Rückstand mit 300 ml Methylenchlorid und 150 ml Wasser versetzt. Die Schichten werden getrennt und die organische Phase noch zweimal mit je 100 ml Wasser ausgeschüttelt. Dann wird die Methylenchloridlösung über Magnesiumsulfat getrocknet und eingedampft. Als Rückstand verbleibt das rohe 4-Chlor-5- cyano-N- (2-diethylamino-ethyl)-2-methoxy-benzamid, das zur Reinigung einmal aus Methylenchlorid/Cyclohexan umkristallisiert wird, Smp. 104–105°.

Das Ausgangsmaterial kann in analoger Weise wie in Beispiel 1 beschrieben hergestellt werden:

N-(2-Amino-ethyl)-4-chlor-5-cyano-2-methoxy-benzamid als farbloses Öl; Hydrochlorid Smp. 227° unter Zersetzung; ausgehend von 120 g (2 Mol) Ethylendiamin in 250 ml Methylenchlorid und 23 g (0,1 Mol) 4-Chlor-5-cyano-2-methoxy-benzoesäurechlorid.

Beispiel 4:

Zu einer Lösung von 1,62 g (6,8 mMol) 2-Methoxy- 4-chlor- 5-cyano-benzoesäureaziridid in 20 ml Toluol wird bei Raumtemperatur unter Rühren 0,63 g (7,2 mMol) N-Ethyl-isopropylamin gegeben. Dann wird auf 100 °C erwärmt und bei dieser Temperatur 10 Std. weitergerührt. Danach lässt man dieses Reaktionsgemisch abkühlen, verdünnt mit 100 ml Methylenchlorid und wäscht zweimal mit 40 ml Wasser. Die vereinigten organischen Phasen werden über Magnesiumsulfat getrocknet, filtriert und anschliessend unter reduziertem Druck zur Trockene eingeengt. Das so erhaltene Rohmaterial wird einmal aus Methylenchlorid/Ether umkristallisiert. Man erhält so 4-Chlor- 5-cyano-N-(2-ethyl-isopropyl-aminoethyl)- 2-methoxy-benzamid mit einem Schmelzpunkt von 103–105 °C.

Das Ausgangsmaterial kann wie folgt hergestellt werden: Zu einer Lösung von 3,9 g (90 mMol) Ethylenimin und 15,3 ml (90 mMol)

Ethyl-isopropylamin in 200 ml Methylenchlorid wird bei −60 °C unter Rühren und Feuchtigkeitsausschluss eine Lösung von 19,7 g (86 mMol) 2-Methoxy- 4-chlor- 5-cyano-benzoesäurechlorid in 200 ml Methylenchlorid im Zeitraum von 15 Minuten zugetropft. Dann lässt man das Reaktionsgemisch auf Raumtemperatur erwärmen und rührt noch 1 Stunde bei dieser Temperatur.

Anschliessend wird das Reaktionsgemisch mit 300 ml Methylenchlorid verdünnt, zweimal mit je 100 ml Wasser gewaschen, die organische Phase mit Magnesiumsulfat getrocknet, filtriert und eingedampft. Das so erhaltene 2-Methoxy-4-chlor-5-cyano-benzoesäureaziridid wird aus Methylenchlorid-Ether umkristallisiert. Es resultiert 4-Chlor-5- cyano-2- methoxy-benzoesäureaziridid vom Smp. 132–134 °C.

Beispiel 5:

In analoger Weise wie in Beispiel 4 beschrieben erhält man 4-Chlor-5-cyano-N-(2-methylethylamino-ethyl)-2-methoxy-benzamid vom Smp. 100–102 °C; ausgehend von 3,6 g (15 mMol) 2-Methoxy-4- chlor-5- cyano-benzoesäureaziridid und 1,4 ml (16 mMol) N-Ethylmethylamin in 20 ml Toluol.

Beispiel 6:

Zu einer Lösung von 4,7 g (12,7 mMol) 4-Chlor- 5-cyano-N- (2-benzylethylamino-ethyl)-2-methoxy-benzamid (vgl. Bsp. 2) und 90 mg (0,6 mMol) Kaliumcarbonat in 200 ml Dichlorethan wird bei 0 °C unter Rühren 2,7 g (19 mMol) α-Chlorethylchloroformat (vgl. J.Org. Chem. 49, 2081 (1984) ) zugetropft. Dann wird 3 Stunden unter Rückfluss erwärmt. Hierauf wird das Reaktionsgemisch im Vakuum zur Trockene eingeengt, der Rückstand mit 200 ml Methanol versetzt und 2 Stunden unter Rückfluss erwärmt. Danach wird erneut eingeengt, der Rückstand in 200 ml 2N Natronlauge aufgenommen und dreimal mit Chloroform extrahiert. Die vereinigten organischen Phasen werden über Magnesiumsulfat getrocknet, filtriert und eingedampft. Das so erhaltene Rohmaterial wird an 200 g Kieselgel mittels Methylenchlorid-Melthanol-Ammoniak im Verhältnis 100:10:1 chromatographisch gereinigt. Die das gewünschte Produkt enthaltenden Fraktionen werden vereinigt und eingedampft. Der Rückstand wird in 50 ml Methanol aufgenommen und unter Kühlung mit Chlorwasserstoffsäure auf pH 3 gestellt. Dann wird Äther zugegeben, wobei das Hydrochlorid auskristallisiert. Man erhält 4-Chlor-5- cyano-N- (2-ethylaminoethyl)-2-methoxy-benzamid-hydrochlorid vom Schmelzpunkt 190–192 °C.

Beispiel 7:

Zu einer Suspension von 3,2 g (10 mMol) 4-Chlor-5- cyano-N- (2-ethylamino-ethyl) -2-methoxy-benzamid-hydrochlor id und 2,8 g (20 mMol) Kaliumcarbonat in 50 ml Ethanol wird 1,23 g (10 mMol) Isopropylbromid gegeben. Dann wird unter Rühren während 12 Stunden zum Rückfluss erwärmt. Man lässt abkühlen, filtriert und engt zur Trockene ein. Der Rückstand wird in Methylenchlorid aufgenommen, dies wird hierauf zweimal mit Wasser gewaschen, über Magnesiumsulfat getrocknet und eingedampft. Nach dem Umkristallisieren aus Methylenchlorid-Ether erhält man 4-Chlor-5-cyano-N-(2-ethyl-isopropylaminoethyl)-2-methoxy-benzamid vom Smp. 103–105 °C.

Beispiel 8:

Analog zu Beispiel 4 erhält man 4-Chlor-5-cyano-N-(2- methyl-tert.-butylamino-ethyl)-2-methoxy-benzamid vom Smp. 137–138,5 °C; ausgehend von 3,6 g (15 mMol) 2-Methoxy-4-chlor-5-cyano-benzoesäureaziridid und 1,4 g (16 mMol) N-tert.-Butylmethylamin.

Beispiel 9:

In analoger Weise wie in Beispiel 4 erhält man 4-Chlor- 5-cyano-N- (2-tert.-butylisopropylamino-ethyl)- 2-methoxy-benzamidhydrochlorid vom Smp. 178–179 °C; ausgehend von 3,6 g (15 mMol) 2-Methoxy-4-chlor-5-cyano-benzoesäureaziridid und 2,6 ml (16 mMol) N-tert.-Butylisopropylamin in 100 ml Toluol.

Beispiel 10:

2,96 g (0,01 Mol) 4-Chlor-5-cyano-N-(2-diethylaminoethyl)-2-hydroxy-benzamid werden zusammen mit 2,06 g (0,015 Mol) Kaliumcarbonat in 100 ml Aceton suspendiert und mit 1,92 g (0,0125 Mol) Diethylsulfat versetzt. Das Reaktionsgemisch wird dann während 5 Stunden zum Sieden erhitzt, dann abgekühlt und die unlöslichen Salze abgenutscht. Das Filtrat wird im Wasserstrahlvakuum eingedampft, der Rückstand in Essigester aufgenommen, zweimal mit Sole gewaschen, über Magnesiumsulfat getrocknet und eingedampft. Als Rückstand verbleibt das 4-Chlor- 5-cyano-N- (2-diethylaminoethyl)-2-ethoxy-benzamid vom Smp. 131,5–133°.

Zur Überführung ins Hydrochlorid wird die Base in Aceton gelöst und mit einer ethanolischen Chlorwasserstofflösung auf deutlich Kongosauer gestellt, wobei das 4-Chlor-5-cyano-N-(2-diethylamino-ethyl)-2-ethoxy-benzamid-hydrochlorid ausfällt. Smp. 179–181°.

Das Ausgangsmaterial kann wie folgt hergestellt werden:

Zu einer Lösung von 23,9 g (0,1 Mol) 4-Chlor-5-cyano-2-methoxybenzoesäure-ethylester in 2 l Methylenchlorid wird unter Rühren und Kühlung bei 15–20° eine Lösung von 37,5 g (0,15 Mol) Bortribromid in 100 ml Methylenchlorid innert 30 Minuten zugetropft. Man lässt dann das Reaktionsgemisch während 15 Stunden weiterrühren und versetzt dann mit 200 ml Eiswasser. Die wässrige Phase wird dann mit gesättigter Sodalösung auf pH 8 gestellt. Der ausgefallene 4-Chlor-5-cyano-salicylsäure-ethylester wird abgenutscht und aus Ethanol umkristallisiert.

22,5 g (0,01 Mol 4-Chlor-5-cyano-salicylsäure-ethylester werden in 110 ml 1 N Natronlauge, 500 ml Ethanol und 500 ml Wasser während 15 Stunden bei Raumtemperatur stehen gelassen.

Dann wird das Ethanol am Rotationsdampfer abdestilliert und die Restlösung mit 60 ml 2 N Salzsäure versetzt, wobei die 4-Chlor-5-cyano-salicylsäure ausfällt. 19,75 g (0,1 Mol) 4-Chlor-5-cyano-salicylsäure werden zusammen mit 200 ml Chloroform und 40 ml Thionylchlorid zum Sieden erhitzt. Nach Beendigung der Gasentwicklung wird am Rotationsverdampfer eingedampft, und das 4-Chlor-5-cyano-N-(2-diethylamino-ethyl)-salicylamid wird in analoger Weise wie in Beispiel 1 beschrieben hergestellt.

Beispiel 11:

Analog zu Beispiel 10 erhält man 4-Chlor-5-cyano-N- (2-diethylamino-ethyl)- 2-n-propoxy-benzamid vom Smp. 88–89°, ausgehend von 2,96 g (0,01 Mol) 4-Chlor-5-cyano-N-(2-diethylaminoethyl)-2-hydroxy-benzamid, 2,06 g (0,015 Mol) Kaliumcarbonat und 1,79 g (0,0105 Mol) n-Propyljodid in 100 ml Aceton; das durch Umsatz mit ethanolischer Chlorwasserstofflösung in Aceton ins Hydrochlorid vom Smp. 164–166° überführt werden kann.

Beispiel 12:

Analog zu Beispiel 10 erhält man 4-Chlor-5-cyano-N- (2-diethylamino-ethyl)- 2-isopropoxy-benzamid vom Smp. 61–62°, ausgehend von 2,96 g (0,01 Mol) 4-Chlor-5-cyano-N-(2-diethylaminoethyl)-2-hydroxy-benzamid, 2,06 g (0,015 Mol) Kaliumcarbonat und 1,79 g (0,0105 Mol) Isopropyljodid in 100 ml Aceton; das durch Umsatz mit ethanolischer Chlorwasserstofflösung in Aceton ins Hydrochlorid vom Smp. 141–142° überführt werden kann.

Beispiel 13:

Zu einer Lösung von 1,16 g (0,01 Mol) 2-Diethylamino-ethylamin in 10 ml Methylenchlorid wird unter Rühren bei 0–5° eine Lösung von 2,3 g (0,01 Mol) 5-Chlor-4-cyano-2-methoxy-benzoesäure-chlorid in 7 ml Methylenchlorid zugetropft, und lässt das Reaktionsgemisch 2 Stunden bei Raumtemperatur rühren. Dann versetzt man mit 10,5 ml 1 N Natronlauge, trennt die Schichten im Scheidetrichter, wäscht die organische Phase zweimal mit Wasser, trocknet sie über Magnesiumsulfat und dampft im Wasserstrahlvakuum ein, wobei das 5-Chlor-4-cyano-N-(2-diethylamino-ethyl)-2-methoxy-benzamid vom Smp. 97–99° erhalten wird.

Zur Überführung ins Hydrochlorid werden 3 g 5-Chlor-4- cyano-N- (2-diethylamino-ethyl)-2-methoxy-benzamid in 20 ml Aceton gelöst und mit einer ethanolischen Chlorwasserstofflösung auf schwach Kongosauer gestellt, wobei das 5-Chlor-4- cyano-N- (2-diethylamino-ethyl)-2-methoxy-benzamid-hydrochlorid ausfällt. Dieses wird abgenutscht und mit wenig Aceton nachgewaschen. Smp. 172–174°.

Das Ausgangsmaterial kann wie folgt hergestellt werden:

6,0 g (0,03 Mol) 4-Chlor-salicylsäure-ethylester werden mit 5 ml Sulfurylchlorid bei Raumtemperatur gerührt, wobei Schwefeldioxid- und Chlorwasserstoffgase entweichen. Nach 2 Stunden und dann nach 4 1/2 Stunden werden je 2 ml Sulfurylchlorid zugegeben und dann 15 Stunden bei Raumtemperatur gerührt. Dann wird das überschüssige Sulfurylchlorid im Wasserstrahlvakuum eingedampft, wobei der rohe 4,5-Dichlorsalicylsäure-ethylester zurückbleibt, das direkt weiterverarbeitet wird.

7,05 g (0,03 Mol) 4,5-Dichlorsalicylsäure-ethylester werden zusammen mit 6,2 g (0,045 Mol) Kaliumcarbonat in 75 ml Aceton suspendiert und unter Rühren mit 4,56 g (0,036 Mol) Dimethylsulfat versetzt. Dann wird während 5 Stunden zum Sieden erhitzt. Nach dem Abkühlen wird von den unlöslichen Salzen abgenutscht und das Filtrat im Wasserstrahlvakuum eingedampft. Dieses wird in Chloroform gelöst, zweimal mit Wasser gewaschen, über Magnesiumsulfat getrocknet und eingedampft. Als Rückstand verbleiben 7,2 g rohes 4,5-Dichlor-2-methoxy-benzoesäure-ethylester, der zur Reinigung an 250 g Kieselgel mit Petrolether/6% Essigester einer Flash Chromatographie unterzogen wird (Fraktionen à 100 ml). Die Fraktionen 12–17 eluierten den reinen 4,5-Dichlor-2-methoxy-benzoesäure-ethylester vom Smp. 48–50°.

2,49 g (0,01 Mol) 4,5-Dichlor-2-methoxy-benzoesäure-ethylester und 1,0 g (0,11 Mol) Kupfer(I)-cyanid werden in 8 ml N-Methyl-2-pyrrolidon unter Rühren während 7 Stunden auf 220° erhitzt. Nach dem Abkühlen giesst man auf Eiswasser und extrahiert mit Essigester, wäscht mit Wasser neutral und trocknet über Magnesiumsulfat. Nach dem Eindampfen des Essigesters verbleiben 2,1 g dunkles Rohprodukt, das zur Reinigung and 150 g Kieselgel mit 8% Essigester einer Flash Chromatographie unterzogen wird. Der 5-Chlor-4-cyano-2-methoxybenzoesäure-ethylester wird in den Fraktionen 30–39 eluiert. Smp. 94–96°.

2,4 g (0,01 Mol) 5-Chlor-4-cyano-2-methoxy-benzoesäure-ethylester werden in 11 ml 1 N Natronlauge, 50 ml Ethanol und 50 ml Wasser während 15 Stunden bei Raumtemperatur stehen gelassen. Dann wird das Ethanol am Rotationsverdampfer abdestilliert und die Restlösung mit 6 ml 2 N Salzsäure versetzt, wobei die 5-Chlor-4-cyano-2-methoxybenzoesäure ausfällt. Diese wird abgenutscht, mit Wasser nachgewaschen und im Exsikkator über Phosphorpentoxid getrocknet. Smp. 220–222°.

2,1 g (0,01 Mol) 5-Chlor-4-cyano-2-methoxy-benzoesäure werden zusammen mit 20 ml Chloroform und 4 ml Thionylchlorid zum Sieden erhitzt. Nachdem keine Gasentwicklung mehr feststellbar ist wird am Rotationsverdampfer eingedampft. Das so erhaltene, rohe 5-Chlor-4-cyano-2-methoxy-benzoesäurechlorid wird direkt weiterverarbeitet.

Beispiel 14:

Analogerweise wie in Beispiel 10 beschrieben kann hergestellt werden:

4- Chlor-5- cyano-2- cyclopentyloxy-N-(2-diethylamino-ethyl)-benzamid, Smp. 161–163°,

ausgehend von 2,96 g (0,01 Mol) 4-Chlor-5-cyano-N- (2-diethylamino-ethyl)-salicylsäureamid, 1,66 g (0,012 Mol) Kaliumcarbonat und 1,57 g (0,0105 Mol) Bromcyclopentan in 50 ml Aceton;

4-Chlor-5-cyano-2-cyclopropyl-methoxy-N-(2-diethylamino-ethyl)-benzamid, Smp. 152–159,5°, ausgehend von 2,96 g (0,01 Mol) 4-Chlor- 5-cyano-N- (2-diethylamino-ethyl)-salicylsäureamid, 1,66 g (0,012 Mol) Kaliumcarbonat und 1,42 g (0,0105 Mol) Cyclopropylmethylbromid in 50 ml Aceton;

2-Allyloxy-4-chlor-5-cyano-N-(2-diethylamino-ethyl)-benzamid, Smp. 145,5–147,5°, ausgehend von 2,96 g (0,01 Mol) 4-Chlor-5-cyano-N-(2-diethylamino-ethyl)-salicylsäureamid, 1,66 g (0,0122 Mol) Kaliumcarbonat und 6,09 g (0,0105 Mol) Allylbromid in 50 ml Aceton; 4-Chlor- 5-cyano-N- (2-diisopropylamino-ethyl)-2-methoxy-benzamid, Smp. 176° (Zers.).

Beispiel 15:

Tabletten, enthaltend 25 mg Wirkstoff, z.B. das 4-Chlor- 5-cyano-N- (2-diethylamino-ethyl)-2-methoxy-benzamid, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung:

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 16:

Tabletten, enthaltend 0,02 g Wirkstoff, z.B. das 4-Chlor- 5-cyano-N- (2-diethylamino-ethyl)-2-methoxy-benzamid, werden wie folgt hergestellt:

| Zusammensetzung (für 10 000 Tabletten) | |
|---|---|
| Wirkstoff | 200,00 g |
| Lactose | 290,80 g |
| Kartoffelstärke | 274,70 g |
| Stearinsäure | 10,00 g |
| Talk | 200,00 g |
| Magnesiumstearat | 2,50 g |
| Kolloidales Siliciumdioxid | 32,00 g |
| Ethanol | q.s. |

Ein Gemisch des Wirkstoffs, der Lactose und 194,70 g Kartoffelstärke wird mit einer ethanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen sein können.

Beispiel 17:

Kapseln, enthaltend 0,025 g des Wirkstoffs, z.B. das 4-Chlor-5-cyano-N-(2-diethylamino-ethyl)-2-methoxy-benzamid, können wie folgt hergestellt werden:

| Zusammensetzung (für 1000 Kapseln) | |
|---|---|
| Wirkstoff | 25,00 g |
| Lactose | 249,00 g |
| Gelatine | 2,00 g |
| Maisstärke | 10,00 g |
| Talk | 15,00 g |
| Wasser | q.s. |

Man mischt den Wirkstoff mit der Lactose, befeuchtet die Mischung gleichmässig mit einer wässrigen Lösung der Gelatine und granuliert sie durch ein Sieb mit einer Maschenweite von 1,2–1,5 mm. Das Granulat mischt man mit der getrockneten Maisstärke und dem Talk und füllt Portionen von 300 mg in Hartgelatinekapseln (Grösse 1) ab.

In analoger Weise wie in den Formulierungsbeispielen 15 bis 17 beschrieben, können entsprechende pharmazeutische Präparate, enthaltend eine Verbindung der Formel I oder ein pharmazeutisch verwendbares Salz davon, insbesondere eine erfindungsgemässe Verbindung gemäss einem der Beispiele 1 bis 14, hergestellt werden.

**Patentansprüche für die Vertragsstaaten: DE, GB, FR, CH, IT, NL, BE, SE, LU**

1. Benzamide der Formel

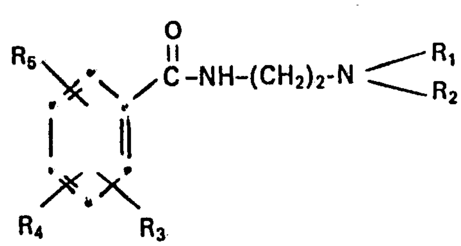

(I)

worin $R_1$ und $R_2$ unabhängig voneinander Niederalkyl bedeuten, $R_3$ Niederalkoxy, $C_3$–$C_5$-Alkenyloxy, $C_3$–$C_7$-Cycloalkoxy oder $C_3$–$C_7$-Cycloalkyl-niederalkoxy bedeutet, $R_4$ Halogen bedeutet und $R_5$ Cyano ist, und deren Salze.

2. Verbindung gemäss Anspruch 1 der Formel I, worin $R_1$ und $R_2$ unabhängig voneinander Niederalkyl mit bis und mit 4 C-Atomen bedeuten, $R_3$ Niederalkoxy mit bis und mit 4 C-Atomen, $C_3$–$C_5$-

Alkenyloxy, $C_3$–$C_7$-Cycloalkoxy oder $C_3$–$C_7$-Cycloalkyl-niederalkoxy mit bis und mit 4 C-Atomen im Niederalkoxyteil bedeutet, $R_4$ Halogen bis und mit Atomnummer 35 bedeutet und $R_5$ Cyano ist, und ihre Salze.

3. Verbindung gemäss Anspruch 1 der Formel I, worin $R_1$ und $R_2$ unabhängig voneinander Niederalkyl mit bis und mit 4 C-Atomen bedeutet, $R_3$ Niederalkoxy mit bis und mit 4 C-Atomen bedeutet, $R_4$ Halogen bis und mit Atomnummer 35 bedeutet und $R_5$ Cyano ist, und ihre Salze.

4. Verbindung gemäss Anspruch 1 der Formel

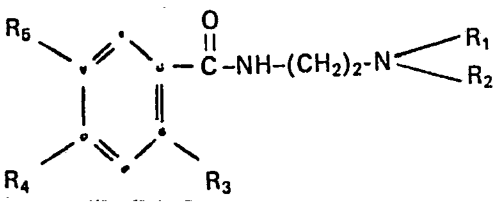

(Ia)

worin $R_1$, $R_2$, $R_3$, $R_4$ und $R_5$ in einem der Ansprüche 1–3 angegebenen Bedeutungen haben, und ihre Salze.

5. Verbindung gemäss Anspruch 1 der Formel Ia, worin $R_1$ und $R_2$ unabhängig voneinander Niederalkyl mit bis und mit 4 C-Atomen bedeutet, $R_3$ Methoxy oder Ethoxy bedeuten, $R_4$ Chlor bedeutet und $R_5$ Cyano ist, und ihre Salze.

6. Verbindung gemäss Anspruch 1 der Formel Ia, worin $R_1$ und $R_2$ Niederalkyl, insbesondere mit bis und mit 4 C-Atomen, bedeutet, $R_3$ Methoxy bedeutet, $R_4$ Chlor bedeutet und $R_5$ Cyano ist, und ihre Salze.

7. Verbindung gemäss einem der Ansprüche 1–6 der Formel I bzw. Ia, worin $R_1$ und $R_2$ unabhängig voneinander Niederalkyl bedeuten, wobei die Summe der C-Atomzahlen 4 bis und mit 6 ausmacht, und ihre Salze.

8. Verbindung gemäss einem der Ansprüche 1–6 der Formel I bzw. Ia, worin $R_1$ und $R_2$ unabhängig voneinander Ethyl oder Isopropyl bedeuten, und ihre Salze.

9. 4-Chlor- 5-cyano-N- (2-diethylamino-ethyl)-2-methoxy-benzamid oder ein Salz davon.

10. 4-Chlor- 5-cyano-N- (2-diethylamino-ethyl)-2-methoxy-benzamid, 4-Chlor-5-cyano-N-(2-ethyl-isopropylamino-ethyl)- 2-methoxy-benzamid, 4-Chlor-5-cyano-N-(2-methylethyl-amino-ethyl)-2-methoxy-benzamid, 4-Chlor-5-cyano-N-(2-methyl-tert.-butylamino-ethyl)-2-methoxy-benzamid, 4- Chlor-5- cyano-N- (2-tert.- butylisopropylamino-ethyl) -2-methoxy-benzamid oder ein Salz davon.

11. 4-Chlor-5-cyano-N-(2-diethylamino-ethyl)-2-ethoxy-benzamid, 4-Chlor-5-cyano-N-(2-diethylamino-ethyl)- 2-n-propoxy-benzamid, 5-Chlor-4- cyano-N- (2-diethylamino-ethyl)-2-methoxy-benzamid, 4-Chlor-5-cyano-N-(2-diisopropylamino-ethyl)-2-methoxy-benzamid, 4-Chlor- 5-cyano-N- (2-diethylamino-ethyl)-2-isopropoxy-benzamid, 4-Chlor-5-cyano- 2-cyclopentyloxy- N- (2-diethylamino-ethyl)-benzamid, 4-Chlor-5-cyano-2-cyclopropyl-methoxy-N- (2-diethylamino-ethyl)-benzamid, 2-Allyloxy-4-chlor-5-cyano-N-(2-diethylamino-ethyl)-benzamid oder ein Salz davon.

12. Verbindung gemäss einem der Ansprüche 1–11 in Form eines pharmazeutisch verwendbaren Säureadditionssalzes.

13. Verbindung gemäss einem der Ansprüche 1–12 zur Anwendung in einem Verfahren zur therapeutischen oder prophylaktischen Behandlung des menschlichen oder tierischen Körpers.

14. Verbindung gemäss einem der Ansprüche 1–13 zur Anwendung in einem Verfahren zur Behandlung von Depressionen und Schizophrenie.

15. Pharmazeutische Präparate, enthaltend eine Verbindung gemäss einem der Ansprüche 1–12.

16. Verwendung von Verbindungen gemäss einem der Ansprüche 1–12 zur Herstellung von Antidepressiva und Arzneimittel zur Behandlung von Schizophrenie.

17. Verfahren zur Herstellung neuer Benzamide der Formel

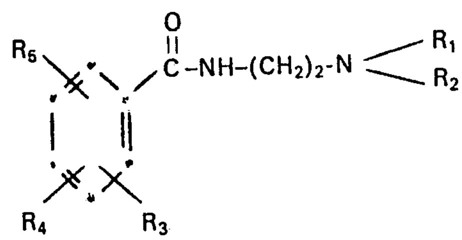

(I)

worin $R_1$ und $R_2$ unabhängig voneinander Niederalkyl bedeuten, $R_3$ Niederalkoxy, $C_3$–$C_5$-Alkenyloxy, $C_3$–$C_7$-Cycloalkoxy oder $C_3$–$C_7$-Cycloalkyl-niederalkoxy bedeutet, $R_4$ Halogen bedeutet und $R_5$ Cyano ist, und deren Salze, dadurch gekennzeichnet, dass man

a) in einer Verbindung der Formel

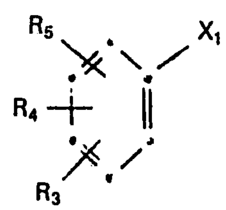

(II)

oder einem Salz davon, worin $X_1$ einen in die Gruppierung der Formel $-CO-NH-(CH_2)_2-N-(R_1)(R_2)$ überführbaren Rest bedeutet, $X_1$ in die Gruppierung $-CO-NH-(CH_2)_2-N(R_1)(R_2)$ überführt, oder

b) eine Verbindung der Formel

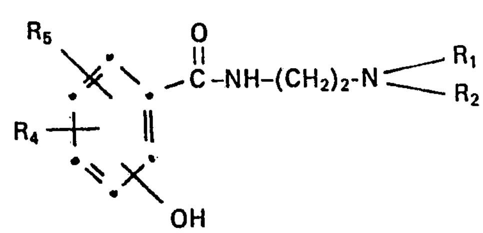

(III)

oder ein Salz davon verethert, oder

c) in einer Verbindung der Formel

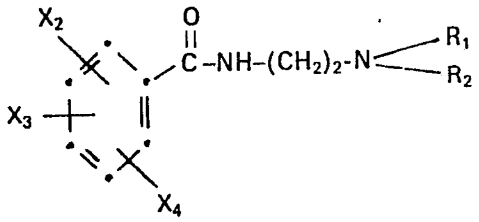

(IV)

oder einem Salz davon, worin einer der Reste $X_2$, $X_3$ und $X_4$ für die Diazoniumgruppierung $-N_2^{\oplus} A^{\ominus}$ und $A^{\ominus}$ für ein Anion steht, und die anderen Reste, entsprechend dem Substitutionsmuster der Verbindung der Formel I, $R_3$, $R_4$ bzw. $R_5$ bedeuten, die Diazoniumgruppe substituiert, oder

d) in einer Verbindung der Formel

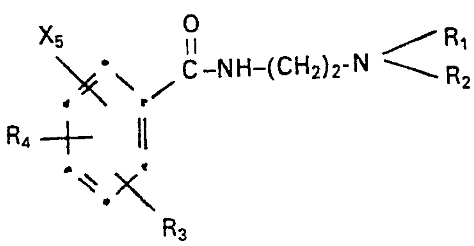

(V)

oder einem Salz davon, worin $X_5$ für einen in Cyano überführbaren Rest steht, $X_5$ in Cyano überführt, und gewünschtenfalls ein verfahrensgemäss erhältliches Salz in die freie Verbindung der Formel I oder in ein anderes Salz überführt und/oder die verfahrensgemäss erhältliche freie Verbindung in ein Salz überführt.

18. Verfahren zur Herstellung pharmazeutischer Präparate gemäss Anspruch 15, dadurch gekennzeichnet, dass man eine Verbindung gemäss einem der Ansprüche 1–12, gegebenenfalls unter Beimengung von üblichen Hilfs- und Trägerstoffen, zu pharmazeutischen Präparaten verarbeitet.

19. Die nach den Verfahren gemäss Anspruch 17 erhältlichen Verbindungen.

**Patentansprüche für den Vertragsstaat AT**

1. Verfahren zur Herstellung neuer Benzamide der Formel

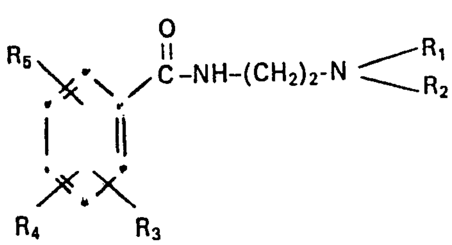

(I)

worin $R_1$ und $R_2$ unabhängig voneinander Niederalkyl bedeuten, $R_3$ Niederalkoxy, $C_3$–$C_5$-Alkenyloxy, $C_3$–$C_7$-Cycloalkoxy oder $C_3$–$C_7$-Cycloalkyl-niederalkoxy bedeutet, $R_4$ Halogen bedeutet und $R_5$ Cyano ist, und deren Salze, dadurch gekennzeichnet, dass man

a) in einer Verbindung der Formel

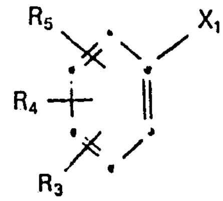

(II)

oder einem Salz davon, worin $X_1$ einen in die Gruppierung der Formel $-CO-NH-(CH_2)_2-N(R_1)(R_2)$ überführbaren Rest bedeutet, $X_1$ in die Gruppierung $-CO-NH-(CH_2)_2-N(R_1)(R_2)$ überführt, oder

b) eine Verbindung der Formel

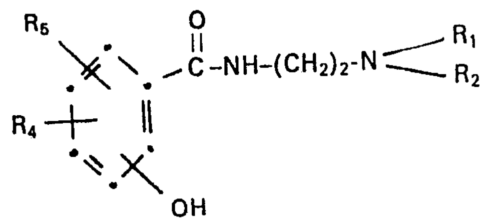

(III)

oder ein Salz davon verethert, oder

c) in einer Verbindung der Formel

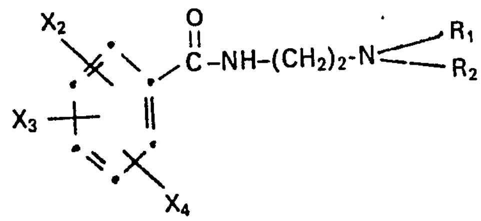

(IV)

oder einem Salz davon, worin einer der Reste $X_2$, $X_3$ und $X_4$ für die Diazoniumgruppierung $-N_2^{\oplus} A^{\ominus}$ und $A^{\ominus}$ für ein Anion steht, und die anderen Reste, entsprechend dem Substitutionsmuster der Verbindung der Formel I, $R_3$, $R_4$ bzw. $R_5$ bedeuten, die Diazoniumgruppe substituiert, oder

d) in einer Verbindung der Formel

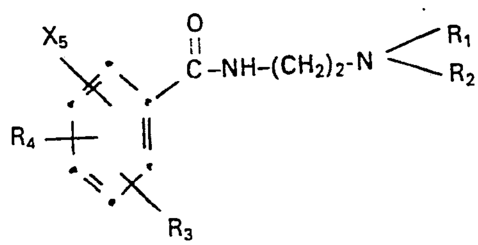

(V)

oder einem Salz davon, worin $X_5$ für einen in Cyano überführbaren Rest steht, $X_5$ in Cyano überführt, und gewünschtenfalls ein verfahrensgemäss erhältliches Salz in die freie Verbindung der Formel I oder in ein anderes Salz überführt und/oder die verfahrensgemäss erhältliche freie Verbindung in ein Salz überführt.

2. Verfahren nach Anspruch 1, dadurch gekennzeichnet, dass man von einer Verbindung der Formel II ausgeht, worin $X_1$ Carboxy oder reaktionsfähiges funktionell abgewandeltes Carboxy bedeutet, und mit einer Verbindung der Formel

$H_2N-(CH_2)_2-N(R_1)(R_2)$ IIa oder einem Salz davon umsetzt.

3. Verfahren nach Anspruch 1, dadurch gekennzeichnet, dass man eine Verbindung der Formel V, worin $X_5$ Hydroxyiminomethyl, O-substituiertes Hydroxyiminomethyl, Carbamoyl, N-monosubstituiertes Carbamoyl bzw. ein entsprechendes Thiocarbamoyl bedeutet, mit einem Dehydrationsmittel behandelt.

4. Verfahren nach einem der Ansprüche 1–3, dadurch gekennzeichnet, dass man eine Verbindung der Formel I, worin $R_1$ und $R_2$ unabhängig voneinander Niederalkyl mit bis und mit 4 C-Atomen bedeuten, $R_3$ Niederalkoxy mit bis und mit 4 C-Atomen, $C_3$–$C_5$-Alkenyloxy, $C_3$–$C_7$-Cycloalkoxy oder $C_3$–$C_7$-Cycloalkyl-niederalkoxy mit bis und mit 4 C-Atomen im Niederalkoxyteil bedeutet, $R_4$ Halogen bis und mit Atomnummer 35 bedeutet und $R_5$ Cyano ist, und ihre Salze herstellt.

5. Verfahren nach einem der Ansprüche 1–3, dadurch gekennzeichnet, dass man eine Verbindung der Formel I, worin $R_1$ und $R_2$ unabhängig voneinander Niederalkyl mit bis und mit 4 C-Atomen bedeutet, $R_3$ Niederalkoxy mit bis und mit 4 C-Atomen bedeutet, $R_4$ Halogen bis und mit Atomnummer 35 bedeutet und $R_5$ Cyano ist, und ihre Salze herstellt.

6. Verfahren nach einem der Ansprüche 1–3, dadurch gekennzeichnet, dass man eine Verbindung der Formel

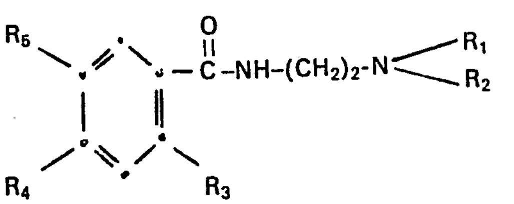

(Ia)

worin $R_1$, $R_2$, $R_3$, $R_4$ und $R_5$ in einem der Ansprüche 1, 4 und 5 angegebenen Bedeutungen haben, und ihre Salze herstellt.

7. Verfahren nach einem der Ansprüche 1–3, dadurch gekennzeichnet, dass man eine Verbindung der Formel Ia, worin $R_1$ und $R_2$ unabhängig voneinander Niederalkyl mit bis und mit 4 C-Atomen bedeutet, $R_3$ Methoxy oder Ethoxy bedeuten, $R_4$ Chlor bedeutet und $R_5$ Cyano ist, und ihre Salze herstellt.

8. Verfahren nach einem der Ansprüche 1–3, dadurch gekennzeichnet, dass man eine Verbindung der Formel Ia, worin $R_1$ und $R_2$ Niederalkyl, insbesondere mit bis und mit 4 C-Atomen, bedeutet, $R_3$ Methoxy bedeutet, $R_4$ Chlor bedeutet und $R_5$ Cyano ist, und ihre Salze herstellt.

9. Verfahren nach einem der Ansprüche 1–3, dadurch gekennzeichnet, dass man eine Verbindung der Formel I bzw. Ia, worin $R_1$ und $R_2$ unabhängig voneinander Niederalkyl bedeuten, wobei die Summe der C-Atomzahlen 4 bis und mit 6 ausmacht, und ihre Salze herstellt.

10. Verfahren nach einem der Ansprüche 1–3, dadurch gekennzeichnet, dass man eine Verbindung der Formel I bzw. Ia, worin $R_1$ und $R_2$ unabhängig voneinander Ethyl oder Isopropyl bedeuten, und ihre Salze herstellt.

11. Verfahren nach einem der Ansprüche 1–3, dadurch gekennzeichnet, dass man 4-Chlor-5-cyano-N- (2-diethylamino-ethyl)- 2-methoxybenzamid oder ein Salz davon herstellt.

12. Verfahren nach einem der Ansprüche 1–3, dadurch gekennzeichnet, dass man 4-Chlor-5-cyano-N- (2-diethylamino-ethyl)- 2-methoxybenzamid, 4-Chlor-5-cyano-N-(2-ethyl-isopropylamino-ethyl)-2-methoxybenzamid, 4-Chlor-5-cyano- N-(2-methylethylamino-ethyl)- 2-methoxybenzamid, 4-Chlor-5-cyano-N-(2-methyl-tert.-butylamino-ethyl)- 2-methoxy-benzamid, 4-Chlor-5-cyano-N-(2-tert.-butylisopropylaminoethyl)-2-methoxy-benzamid oder ein Salz davon herstellt.

13. Verfahren nach einem der Ansprüche 1–3, dadurch gekennzeichnet, dass man 4-Chlor-5-cyano- N- (2-diethylamino- ethyl)- 2-ethoxybenzamid, 4-Chlor-5-cyano-N-(2-diethylaminoethyl)- 2-n-propoxy-benzamid, 5- Chlor- 4-cyano- N- (2-diethylamino- ethyl)- 2-methoxy-benzamid, 4-Chlor-5-cyano-N-(2-diisopropylamino-ethyl)-2-methoxy-benzamid, 4-Chlor-5-cyano-N-(2-diethylamino-ethyl)-2-isopropoxybenzamid, 4-Chlor- 5-cyano-2-cyclopentyloxy-N-(2-diethylamino-ethyl)-benzamid, 4-Chlor-5-cyano-2-cyclopropyl-methoxy-N-(2-diethylamino-ethyl)-benzamid, 2-Allyloxy- 4-chlor-5-cyano-N-(2-diethylamino-ethyl)-benzamid oder ein Salz davon herstellt.

14. Verfahren nach einem der Ansprüche 1–3, dadurch gekennzeichnet, dass man eine Verbindung der Formel I in Form eines pharmazeutisch verwendbaren Säureadditionssalzes herstellt.

15. Verfahren zur Herstellung pharmazeutischer Präparate, dadurch gekennzeichnet, dass man eine Verbindung gemäss einem der Ansprüche 1 und 4–14, gegebenenfalls unter Beimengung von üblichen Hilfs- und Trägerstoffen, zu pharmazeutischen Präparaten verarbeitet.

**Revendications pour les Etats Contractants: BE, CH, DE, FR, GB, IT, LI, LU, NL, SE**

1. Benzamides de formule:

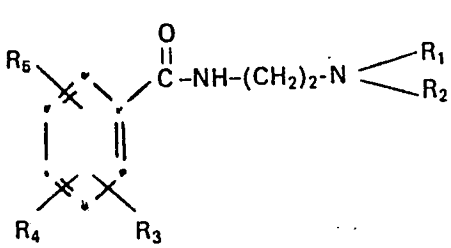

(I)

dans laquelle $R_1$ et $R_2$ représentent chacun, indépendamment l'un de l'autre, un groupe alkyle inférieur, $R_3$ représente un groupe alcoxy inférieur, alcényloxy en $C_3$–$C_5$, cycloalcoxy en $C_3$–$C_7$ ou (cycloalkyle en $C_3$–$C_7$)-alcoxy inférieur, $R_4$ représente un halogène et $R_5$ un groupe cyano, et leurs sels.

2. Composé selon la revendication 1, répondant à la formule I dans laquelle $R_1$ et $R_2$ représentent

chacun, indépendamment l'un de l'autre un groupe alkyle inférieur contenant jusqu'à 4 atomes de carbone inclus, $R_3$ représente un groupe alcoxy inférieur contenant jusqu'à 4 atomes de carbone inclus, un groupe alcényloxy en $C_3$–$C_5$, cycloalcoxy en $C_3$–$C_7$ ou (cycloalkyle en $C_3$–$C_7$)-alcoxy inférieur contenant jusqu'à 4 atomes de carbone inclus dans la partie alcoxy inférieur, $R_4$ représente un halogène de numéro atomique allant jusqu'à 35 et $R_5$ un groupe cyano, et ses sels.

3. Composé selon la revendication 1, répondant à la formule I dans laquelle $R_1$ et $R_2$ représentent chacun, indépendamment l'un de l'autre, un groupe alkyle inférieur contenant jusqu'à 4 atomes de carbone inclus, $R_3$ représente un groupe alcoxy inférieur contenant jusqu'à 4 atomes de carbone inclus, $R_4$ représente un halogène de numéro atomique allant jusqu'à 35 inclus et $R_5$ un groupe cyano, et ses sels.

4. Composé selon la revendication 1, répondant à la formule:

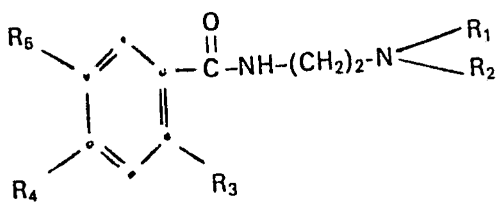

(Ia)

dans laquelle $R_1$, $R_2$, $R_3$, $R_4$ et $R_5$ ont les significations indiquées dans l'une des revendications 1 à 3, et ses sels.

5. Composé selon la revendication 1, répondant à la formule Ia dans laquelle $R_1$ et $R_2$ représentent chacun, indépendamment l'un de l'autre un groupe alkyle inférieur contenant jusqu'à 4 atomes de carbone inclus, $R_3$ représente un groupe méthoxy ou éthoxy, $R_4$ représente le chlore et $R_5$ un groupe cyano, et ses sels.

6. Composé selon la revendication 1, répondant à la formule Ia dans laquelle $R_1$ et $R_2$ représentent des groupes alkyle inférieurs, contenant en particulier jusqu'à 4 atomes de carbone inclus, $R_3$ représente un groupe méthoxy, $R_4$ représente le chlore et $R_5$ un groupe cyano, et ses sels.

7. Composé selon l'une des revendications 1 à 6, répondant à la formule I ou Ia dans lesquelles $R_1$ et $R_2$ représentent chacun, indépendamment l'un de l'autre, un groupe alkyle inférieur, à des nombres d'atomes de carbone dont la somme va de 4 à 6 inclus, et ses sels.

8. Composé selon l'une des revendications 1 à 6, répondant à la formule I ou Ia dans lesquelles $R_1$ et $R_2$ représentent chacun, indépendamment l'un de l'autre, un groupe éthyle ou isopropyle, et ses sels.

9. Le 4-chloro-5-cyano-N-(2-diéthylaminoéthyl)-2-méthoxybenzamide ou un sel de ce composé.

10. Le 4-chloro-5-cyano-N-(2-diéthylaminoéthyl)-2-méthoxybenzamide, le 4-chloro-5-cyano-N- (2-éthylisopropylaminoéthyl)- 2-méthoxybenzamide, le 4-chloro-5-cyano-N-(2-méthyléthylaminoéthyl)- 2-méthoxybenzamide, le 4-chloro- 5-cyano-N- (2-méthyltertbutylaminoéthyl)-2-méthoxybenzamide, le 4-chloro-5-cyano-N- (2-tertbutylisopropylaminoéthyl)- 2-méthoxybenzamide ou un sel de l'un de ces composés.

11. Le 4-chloro-5-cyano-N-(2-diéthylaminoéthyl)-2-éthoxybenzamide, le 4-chloro-5-cyano-N- (2-diéthylaminoéthyl)- 2-n-propoxybenzamide, le 5-chloro-4-cyano-N-(2-diéthylaminoéthyl)-2-méthoxybenzamide, le 4-chloro-5-cyano-N- (2-diisopropylaminoéthyl)- 2-méthoxybenzamide, le 4- chloro- 5-cyano-N- (2-diéthylaminoéthyl)-2-isopropoxybenzamide, le 4-chloro- 5-cyano- 2-cyclopentyloxy-N- (2-diéthylaminoéthyl)-benzamide, le 4-chloro-5-cyano-2-cyclopropylméthoxy-N- (2-diéthylaminoéthyl)-benzamide, le 2-allyloxy-4-chloro-5-cyano-N-(2-diéthylaminoéthyl)-benzamide ou un sel de l'un de ces composés.

12. Composé selon l'une des revendications 1 à 11, à l'état de sel formé par addition avec un acide acceptable pour l'usage pharmaceutique.

13. Composé selon l'une des revendications 1 à 12, pour l'utilisation dans un procédé pour le traitement thérapeutique ou prophylactique de l'organisme humain ou animal.

14. Composé selon l'une des revendications 1 à 13, pour l'utilisation dans un procédé pour le traitement des dépressions et de la schizophrénie.

15. Compositions pharmaceutiques contenant un composé selon l'une des revendications 1 à 12.

16. Utilisation des composés selon l'une des revendications 1 à 12 pour la préparation de médicaments antidépressifs et de médicaments pour le traitement de la schizophrénie.

17. Procédé de préparation de nouveaux benzamides de formule:

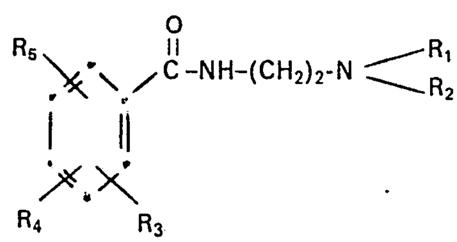

(I)

dans laquelle $R_1$ et $R_2$ représentent chacun, indépendamment l'un de l'autre un groupe alkyle inférieur, $R_3$ représente un groupe alcoxy inférieur, alcényloxy en $C_3$–$C_5$, cycloalcoxy en $C_3$–$C_7$ ou (cycloalkyle en $C_3$–$C_7$)-alcoxy inférieur, $R_4$ représente un halogène et $R_5$ un groupe cyano, et de leurs sels, caractérisé en ce que:

(a) dans un composé de formule:

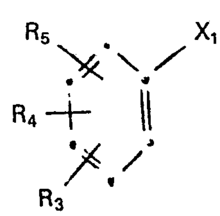

(II)

ou un sel de ce composé, dans lesquels $X_1$ représente un groupe convertible en le groupement de formule $-CO-NH-(CH_2)_2-N(R_1)(R_2)-$, on convertit $X_1$ en le groupement $-CO-NH-(CH_2)_2-N-(R_1)(R_2)-$, ou bien

(b) on éthérifie un composé de formule:

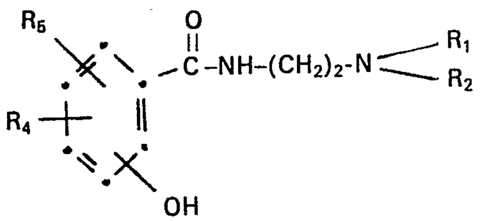

(III)

ou un sel d'un tel composé, ou bien

(c) dans un composé de formule:

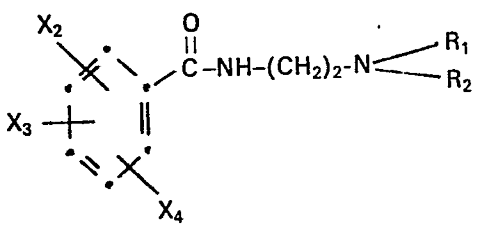

(IV)

ou un sel d'un tel composé, dans lesquels l'un des symboles $X_2$, $X_3$ et $X_4$ représentent le groupement diazonium $-N_2^{\oplus} A^{\ominus}$ et $A^{\ominus}$ représente un anion, et les autres symboles, selon le modèle de substitution du composé de formule I, représentent respectivement $R_3$, $R_4$ ou $R_5$, on substitue le groupe diazonium, ou bien

(d) dans un composé de formule:

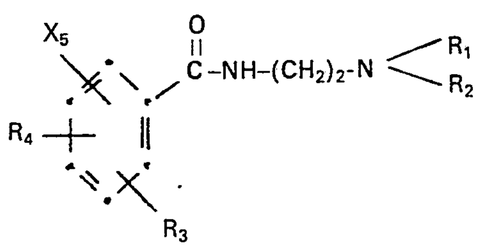

(V)

ou un sel d'un tel composé, dans lesquels $X_5$ représente un groupe convertible en un groupe cyano, on convertit $X_5$ en un groupe cyano et si on le désire, on convertit un sel ainsi obtenu en le composé libre de formule I ou en un autre sel et/ou on convertit un composé libre ainsi obtenu en un sel.

18. Procédé de préparation des compositions pharmaceutiques selon la revendication 15, caractérisé en ce que l'on met un composé selon l'une des revendications 1 à 12, le cas échéant avec adjonction de produits auxiliaires et véhicules usuels, à l'état de compositions pharmaceutiques.

19. Les composés qu'on peut obtenir par les procédés de la revendication 17.

**Revendications pour l'Etat Contractant: AT**

1. Procédé de préparation de nouveaux benzamides de formule

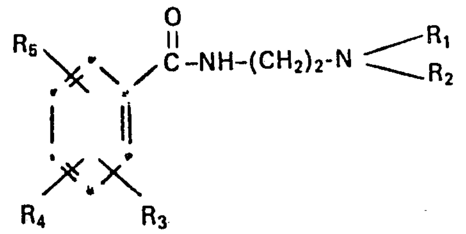

(I)

dans laquelle $R_1$ et $R_2$ représentent chacun, indépendamment l'un de l'autre, un groupe alkyle inférieur, $R_3$ représente un groupe alcoxy inférieur, alcényloxy en $C_3$–$C_5$, cycloalcoxy en $C_3$–$C_7$ ou (cycloalkyle en $C_3$–$C_7$)-alcoxy inférieur, $R_4$ représente un halogène et $R_5$ un groupe cyano, et de leurs sels, caractérisé en ce que

a) dans un composé de formule

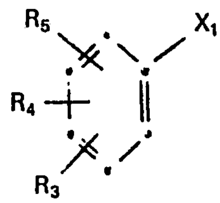

(II)

ou un sel d'un tel composé, dans lesquels $X_1$ représente un substituant convertible en le groupement de formule $-CO-NH-(CH_2)_2-N(R_1)(R_2)$, on convertit $X_1$ en le groupement $-CO-NH-(CH_2)_2-N(R_1)(R_2)$, ou bien

b) on éthérifie un composé de formule

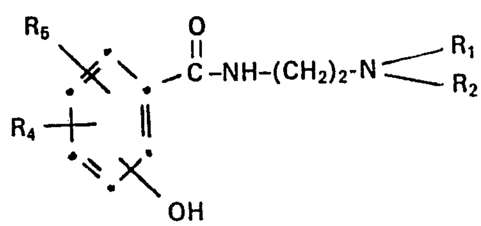

(III)

ou un sel d'un tel composé, ou bien

c) dans un composé de formule

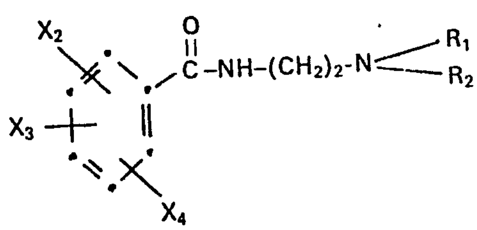

(IV)

ou un sel de ce composé, dans lesquels l'un des symboles $X_2$, $X_3$ et $X_4$ représente le groupement diazonium $-N_2^{\oplus} A^{\ominus}$ et $A^{\ominus}$ représente un anion, et les autres symboles, selon le modèle de substitution du composé de formule I, représentent $R_3$, $R_4$ ou respectivement $R_5$, on substitue le groupe diazonium, ou bien

d) dans un composé de formule

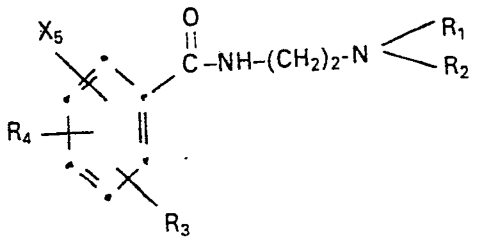

(V)

ou un sel de ce composé, dans lesquels $X_5$ représente un substituant convertible en un groupe cyano, on convertit $X_5$ en un groupe cyano et si on le désire, on convertit un sel ainsi obtenu en le composé libre de formule I ou en un autre sel et/ou on convertit un composé libre ainsi obtenu en un sel.

2. Procédé selon la revendication 1, caractérisé en ce que l'on part d'un composé de formule II dans laquelle $X_1$ représente un groupe carboxy ou un groupe carboxy qui a subi une modification fonctionnelle et qui est réactif, et on le fait réagir avec un composé de formule $H_2N-(CH_2)_2-N(R_1)(R_2)$ IIa ou avec un sel d'un tel composé.

3. Procédé selon la revendication 1 caractérisé en ce que l'on traite par un agent déshydratant un composé de formule V dans laquelle $X_5$ représente un groupe hydroxyiminométhyle, hydroxyiminométhyle substitué sur l'oxygène, carbamoyle, carbamoyle monosubstitué à l'azote ou un groupe thiocarbamoyle correspondant.

4. Procédé selon l'une des revendications 1 à 3, caractérisé en ce que l'on prépare un composé de formule I dans laquelle $R_1$ et $R_2$ représentent chacun, indépendamment l'un de l'autre,un groupe alkyle inférieur contenant jusqu'à 4 atomes de carbone inclus, $R_3$ représente un groupe alcoxy inférieur contenant jusqu'à 4 atomes de carbone inclus, un groupe alcényloxy en $C_3$–$C_5$, cycloalcoxy en $C_3$–$C_7$ ou (cycloalkyle en $C_3$–$C_7$)-alcoxy inférieur contenant jusqu'à 4 atomes de carbone inclus dans la partie alcoxy inférieur, $R_4$ représente un halogène de numéro atomique allant jusqu'à 35 inclus et $R_5$ représente un groupe cyano, et ses sels.

5. Procédé selon l'une des revendications 1 à 3, caractérisé en ce que l'on prépare un composé de formule I dans laquelle $R_1$ et $R_2$ représentent chacun, indépendamment l'un de l'autre, un groupe alkyle inférieur contenant jusqu'à 4 atomes de carbone inclus, $R_3$ représente un groupe alcoxy inférieur contenant jusqu'à 4 atomes de carbone inclus, $R_4$ représente un halogène de numéro atomique allant jusqu'à 35 inclus et $R_5$ un groupe cyano, et ses sels.

6. Procédé selon l'une des revendications 1 à 3, caractérisé en ce que l'on prépare un composé de formule

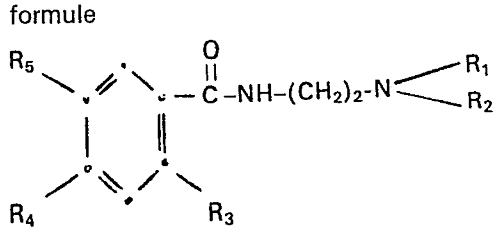

(Ia)

dans laquelle $R_1$, $R_2$, $R_3$, $R_4$ et $R_5$ ont les significations indiquées dans l'une des revendications 1, 4 et 5, et ses sels.

7. Procédé selon l'une des revendications 1 à 3, caractérisé en ce que l'on prépare un composé de formule Ia dans laquelle $R_1$ et $R_2$ représentent chacun, indépendamment l'un de l'autre, un groupe alkyle inférieur contenant jusqu'à 4 atomes de carbone inclus, $R_3$ représente un groupe méthoxy ou éthoxy, $R_4$ représente le chlore et $R_5$ un groupe cyano, et ses sels.

8. Procédé selon l'une des revendications 1 à 3, caractérisé en ce que l'on prépare un composé de formule Ia dans laquelle $R_1$ et $R_2$ représentent des groupes alkyle inférieurs, en particulier des groupes alkyle inférieurs contenant jusqu'à 4 atomes de carbone inclus, $R_3$ représente un groupe méthoxy, $R_4$ représente le chlore et $R_5$ un groupe cyano, et ses sels.

9. Procédé selon l'une des revendications 1 à 3, caractérisé en ce que l'on prépare un composé de formule I ou Ia dans lesquelles $R_1$ et $R_2$ représentent chacun, indépendamment l'un de l'autre, un groupe alkyle inférieur, avec des nombres d'atomes de carbone dont la somme va de 4 à 6 inclus, et ses sels.

10. Procédé selon l'une des revendications 1 à 3 caractérisé en ce que l'on prépare un composé de formule I ou Ia dans lesquelles $R_1$ et $R_2$ représentent chacun, indépendamment l'un de l'autre, un groupe éthyle ou isopropyle, et ses sels.

11. Procédé selon l'une des revendications 1 à 3, caractérisé en ce que l'on prépare le 4-chloro-5-cyano-N- (2-diéthylaminoéthyl)- 2-méthoxybenzamide ou un sel de ce composé.

12. Procédé selon l'une des revendications 1 à 3, caractérisé en ce que l'on prépare, le 4-chloro-5-cyano-N-(2-diéthylaminoéthyl)-2-méthoxybenzamide, le 4-chloro-5-cyano-N-(2-éthylisopropylaminoéthyl)- 2-méthoxybenzamide, le 4-chloro- 5-cyano-N- (2-méthyléthylaminoéthyl)-2-méthoxybenzamide, le 4-chloro-5-cyano-N- (2-méthyltertbutylaminoéthyl) -2-méthoxybenzamide, le 4-chloro-5-cyano-N-(2-tertbutylisopropylaminoéthyl)- 2-méthoxybenzamide ou un sel de l'un de ces composés, ou un sel de l'un de ces composés.

13. Procédé selon l'une des revendications 1 à 3, caractérisé en ce que l'on prépare, le 4-chloro-5- cyano-N- (2-diéthylaminoéthyl)- 2-éthoxybenzamide, le 4-chloro-5-cyano-N-(2-diéthylaminoéthyl)-2-n-propoxybenzamide, le 5-chloro-4-cyano-N-(2-diéthylaminoéthyl)-2-méthoxybenzamide, le 4-chloro-5-cyano-N-(2-diisopropylaminoéthyl)-2-méthoxybenzamide, le 4-chloro- 5-cyano-N- (2-diéthylaminoéthyl)-2-isopropoxybenzamide, le 4-chloro-5-cyano-2-cyclopentyloxy-N-(2-diéthylaminoéthyl)-benzamide, le 4-chloro-5-cyano-2-cyclopropylméthoxy-N-(2-diéthylaminoéthyl)-benzamide, le 2-allyloxy-4-chloro-5-cyano-N-(2-diéthylaminoéthyl)-benzamide, ou un sel de l'un de ces composés.

14. Procédé selon l'une des revendications 1 à 3, caractérisé en ce que l'on prépare un composé

de formule I à l'état de sel formé par addition avec un acide acceptable pour l'usage pharmaceutique.

15. Procédé de préparation de compositions pharmaceutiques, caractérisé en ce que l'on met un composé selon l'une des revendications 1 et 4 à 14, le cas échéant avec adjonction de produits auxiliaires et véhicules usuels, à l'état de composition pharmaceutique.

**Claims for the Contracting States: BE, CH, DE, FR, GB, IT, LI, LU, NL, SE**

1. Benzamides of the formula

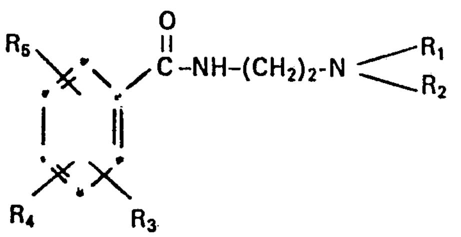

(I)

in which $R_1$ and $R_2$, independently of one another, each represents lower alkyl, $R_3$ represents lower alkoxy, $C_3$–$C_5$-alkenyloxy, $C_3$–$C_7$-cycloalkoxy or $C_3$–$C_7$-cycloalkyl-lower alkoxy, $R_4$ represents halogen and $R_5$ represents cyano, and their salts.

2. A compound according to claim 1 of the formula I in which $R_1$ and $R_2$, independently of one another, each represents lower alkyl having up to and including 4 carbon atoms, $R_3$ represents lower alkoxy having up to and including 4 carbon atoms, $C_3$–$C_5$-alkenyloxy, $C_3$–$C_7$-cycloalkoxy or $C_3$–$C_7$-cycloalkyl-lower alkoxy having up to and including 4 carbon atoms in the lower alkoxy moiety, $R_4$ represents halogen having an atomic number of up to and including 35 and $R_5$ represents cyano, and their salts.

3. A compound according to claim 1 of the formula I in which $R_1$ and $R_2$, independently of one another, each represents lower alkyl having up to and including 4 carbon atoms, $R_3$ represents lower alkoxy having up to and including 4 carbon atoms, $R_4$ represents halogen having an atomic number of up to and including 35 and $R_5$ represents cyano, and their salts.

4. A compound according to claim 1 of the formula

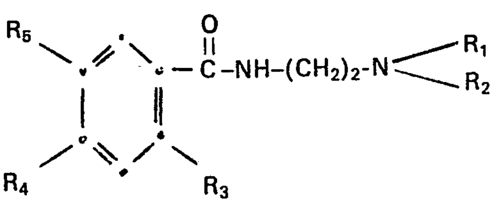

(Ia)

in which $R_1$, $R_2$, $R_3$, $R_4$ and $R_5$ have the meanings given in any one of claims 1 to 3, and their salts.

5. A compound according to claim 1 of the formula Ia in which $R_1$ and $R_2$, independently of one another, each represents lower alkyl having up to and including 4 carbon atoms, $R_3$ represents methoxy or ethoxy, $R_4$ represents chlorine and $R_5$ represents cyano, and their salts.

6. A compound according to claim 1 of the formula Ia in which $R_1$ and $R_2$ represent lower alkyl, especially lower alkyl having up to and including 4 carbon atoms, $R_3$ represents methoxy, $R_4$ represents chlorine and $R_5$ represents cyano, and their salts.

7. A compound according to any one of claims 1 to 6 of the formula I or Ia in which $R_1$ and $R_2$, independently of one another, each represents lower alkyl, the sum of the number of carbon atoms in the lower alkyl radicals being from 4 up to and including 6, and their salts.

8. A compound according to any one of claims 1 to 6 of the formula I or Ia in which $R_1$ and $R_2$, independently of one another, each represents ethyl or isopropyl, and their salts.

9. 4-chloro- 5-cyano-N- (2-diethylaminoethyl)-2-methoxybenzamide or a salt thereof.

10. 4-chloro- 5-cyano-N- (2-diethylaminoethyl)-2-methoxybenzamide, 4-chloro-5-cyano-N- (2-ethylisopropylaminoethyl)- 2-methoxybenzamide, 4-chloro- 5-cyano-N- (2-methylethylaminoethyl)- 2-methoxybenzamide, 4-chloro-5-cyano-N- (2-methyl-tert.-butylaminoethyl)- 2-methoxybenzamide, 4-chloro-5-cyano-N- (2-tert.-butylisopropylaminoethyl)-2-methoxybenzamide or a salt thereof.

11. 4-chloro- 5-cyano-N- (2-diethylaminoethyl)-2-ethoxybenzamide, 4-chloro-5-cyano-N-(2-diethylaminoethyl)- 2-n-propoxybenzamide, 5-chloro-4-cyano-N-(2-diethylaminoethyl)-2-methoxybenzamide, 4-chloro-5-cyano-N-(2-diisopropylaminoethyl)- 2-methoxybenzamide, 4-chloro- 5-cyano-N- (2-diethylaminoethyl)-2-isopropoxybenzamide, 4-chloro-5-cyano-2-cyclopentyloxy-N- (2-diethylaminoethyl)-benzamide, 4-chloro- 5-cyano- 2-cyclopropylmethoxy-N- (2-diethylaminoethyl)-benzamide, 2-allyloxy- 4-chloro- 5-cyano-N-(2-diethylaminoethyl)-benzamide or a salt thereof.

12. A compound according to any one of claims 1 to 11 in the form of a pharmaceutically acceptable acid addition salt.

13. A compound according to any one of claims 1 to 12 for use in a method for the therapeutic or prophylactic treatment of the human or animal body.

14. A compound according to any one of claims 1 to 13 for use in a method for the treatment of depression and schizophrenia.

15. Pharmaceutical preparations containing a compound according to any one of claims 1 to 12.

16. The use of compounds according to any one of claims 1 to 12 for the manufacture of antidepressants and medicaments for the treatment of schizophrenia.

17. A process for the manufacture of novel benzamides of the formula

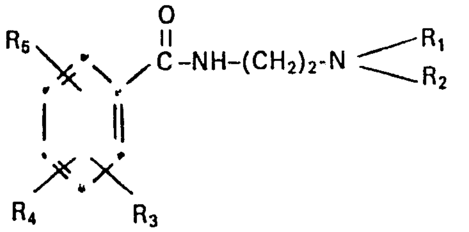

(I)

in which $R_1$ and $R_2$, independently of one another, each represents lower alkyl, $R_3$ represents lower alkoxy, $C_3$–$C_5$-alkenyloxy, $C_3$–$C_7$-cycloalkoxy or $C_3$–$C_7$-cycloalkyl-lower alkoxy, $R_4$ represents halogen and $R_5$ represents cyano, and their salts, characterised in that

a) in a compound of the formula

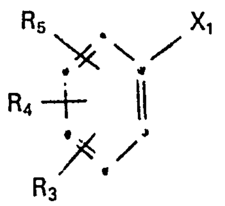

(II)

or in a salt thereof, in which $X_1$ represents a radical that can be converted into the grouping of the formula $-CO-NH-(CH_2)_2-N(R_1)(R_2)$, $X_1$ is converted into the grouping $-CO-NH-(CH_2)_2-N(R_1)(R_2)$, or

b) a compound of the formula

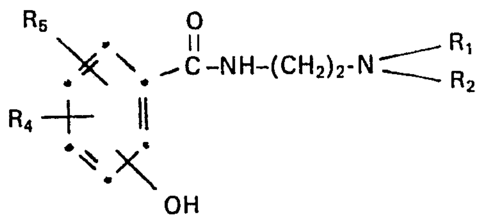

(III)

or a salt thereof, is etherified, or

c) in a compound of the formula

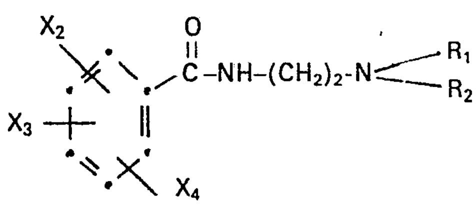

(IV)

or in a salt thereof, in which one of the radicals $X_2$, $X_3$ and $X_4$ represents the diazonium grouping $-N_2^{\oplus}$ $A^{\ominus}$ and $A^{\ominus}$ represents an anion, and the other radicals represent $R_3$, $R_4$ or $R_5$ corresponding to the substitution pattern of the compound of the formula I, the diazonium group is substituted, or

d) in a compound of the formula

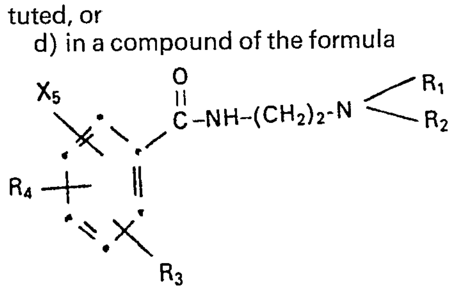

(V)

or in a salt thereof, in which $X_5$ represents a radical that can be converted into cyano, $X_5$ is converted into cyano, and, if desired, a salt obtainable according to the process is converted into the free compound of the formula I or into a different salt and/or the free compound obtainable acćording to the process is converted into a salt.

18. A process for the manufacture of pharmaceutical preparations according to claim 15, characterised in that a compound according to any one of claims 1 to 12, optionally with the admixture of customary adjuncts and carriers, is processed to form pharmaceutical preparations.

19. The compounds obtainable by the processes according to claim 17.

**Claims for the Contracting State: AT**

1. A process for the manufacture of novel benzamides of the formula

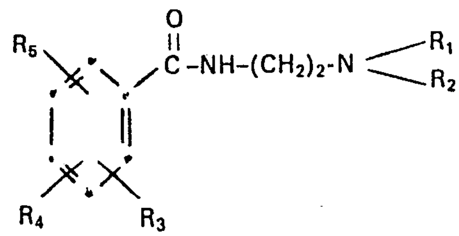

(I)

in which $R_1$ and $R_2$, independently of one another, each represents lower alkyl, $R_3$ represents lower alkoxy, $C_3$–$C_5$-alkenyloxy, $C_3$–$C_7$-cycloalkoxy or $C_3$–$C_7$-cycloalkyl-lower alkoxy, $R_4$ represents halogen and $R_5$ represents cyano, and their salts, characterised in that

a) in a compound of the formula

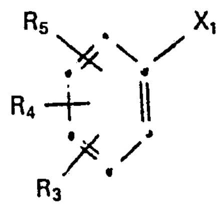

(II)

or in a salt thereof, in which $X_1$ represents a radical that can be converted into the grouping of the formula $-CO-NH-(CH_2)_2-N(R_1)(R_2)$, $X_1$ is converted into the grouping $-CO-NH-(CH_2)_2-N(R_1)(R_2)$, or

b) a compound of the formula

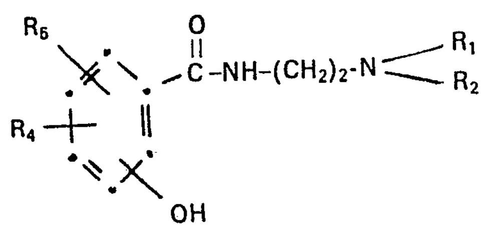

(III)

or a salt thereof, is etherified, or

c) in a compound of the formula

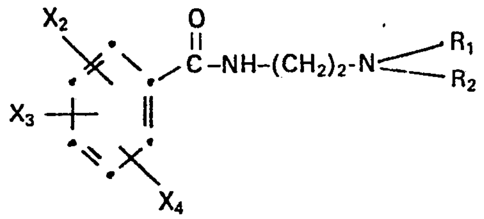

(IV)

or in a salt thereof, in which one of the radicals $X_2$, $X_3$ and $X_4$ represents the diazonium grouping $-N_2^{\oplus}$ $A^{\ominus}$ and $A^{\ominus}$ represents an anion, and the other radicals represent $R_3$, $R_4$ or $R_5$ corresponding to the substitution pattern of the compound of the formula I, the diazonium group is substituted, or

d) in a compound of the formula

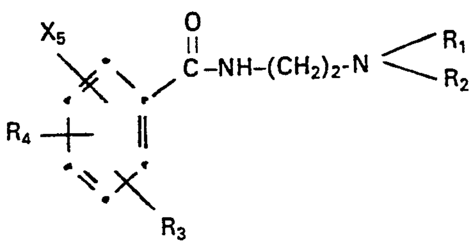

(V)

or in a salt thereof, in which $X_5$ represents a radical that can be converted into cyano, $X_5$ is converted into cyano, and, if desired, a salt obtainable according to the process is converted into the free compound of the formula I or into a different salt and/or the free compound obtainable according to the process is converted into a salt.

2. A process according to claim 1, characterised in that a compound of the formula II in which $X_1$ represents carboxy or reactive functionally modified carboxy is used as starting material and is reacted with a compound of the formula $H_2N-(CH_2)_2-N(R_1)(R_2)$ IIa or with a salt thereof.

3. A process according to claim 1, characterised in that a compound of the formula V in which $X_5$ represents hydroxyiminomethyl, O-substituted hydroxyiminomethyl, carbamoyl, N-monosubstituted carbamoyl or a corresponding thiocarbamoyl, is treated with a dehydration agent.

4. A process according to any one of claims 1 to 3, characterised in that a compound of the formula I in which $R_1$ and $R_2$, independently of one another, each represents lower alkyl having up to and including 4 carbon atoms, $R_3$ represents lower alkoxy having up to and including 4 carbon atoms, $C_3$–$C_5$-alkenyloxy, $C_3$–$C_7$-cycloalkoxy or $C_3$–$C_7$-cycloalkyl-lower alkoxy having up to and including 4 carbon atoms in the lower alkoxy moiety, $R_4$ represents halogen having an atomic number of up to and including 35 and $R_5$ represents cyano, and its salts, are manufactured.

5. A process according to any one of claims 1 to 3, characterised in that a compound of the formula I in which $R_1$ and $R_2$, independently of one another, each represents lower alkyl having up to and including 4 carbon atoms, $R_3$ represents lower alkoxy having up to and including 4 carbon atoms, $R_4$ represents halogen having an atomic number of up to and including 35 and $R_5$ represents cyano, and its salts, are manufactured.

6. A process according to any one of claims 1 to 3, characterised in that a compound of the formula

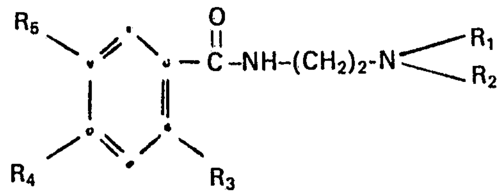

(Ia)

in which $R_1$, $R_2$, $R_3$, $R_4$ and $R_5$ have the meanings given in any one of claims 1, 4 and 5, and its salts, are manufactured.

7. A process according to any one of claims 1 to 3, characterised in that a compound of the formula Ia in which $R_1$ and $R_2$, independently of one another, each represents lower alkyl having up to and including 4 carbon atoms, $R_3$ represents methoxy or ethoxy, $R_4$ represents chlorine and $R_5$ represents cyano, and its salts, are manufactured.

8. A process according to any one of claims 1 to 3, characterised in that a compound of the formula Ia in which $R_1$ and $R_2$ represent lower alkyl, especially lower alkyl having up to and including 4 carbon atoms, $R_3$ represents methoxy, $R_4$ represents chlorine and $R_5$ represents cyano, and its salts, are manufactured.

9. A process according to any one of claims 1 to 3, characterised in that a compound of the formula I or Ia in which $R_1$ and $R_2$, independently of one another, each represents lower alkyl, the sum of the number of carbon atoms in the lower alkyl radicals being from 4 up to and including 6, and its salts, are manufactured.

10. A process according to any one of claims 1 to 3, characterised in that a compound of the formula I or Ia in which $R_1$ and $R_2$, independently of one another each represents ethyl or isopropyl, and its salts, are manufactured.

11. A process according to any one of claims 1 to 3, characterised in that 4-chloro-5-cyano-N-(2-diethylaminoethyl)-2-methoxybenzamide or a salt thereof is manufactured.

12. A process according to any one of claims 1 to 3, characterised in that 4-chloro-5-cyano-N-(2-diethylaminoethyl)- 2-methoxybenzamide, 4-chloro- 5-cyano-N- (2-ethylisopropylaminoethyl)- 2-methoxybenzamide, 4-chloro- 5-cyano-N- (2-methylethylaminoethyl)- 2-methoxybenzamide, 4-chloro- 5-cyano-N- (2-methyl-tert.-butylaminoethyl)-2-methoxybenzamide, 4-chloro-5-cyano-N-(2-tert.-butylisopropylaminoethyl)-2-methoxybenzamide or a salt thereof is manufactured.

13. A process according to any one of claims 1 to 3, characterised in that 4-chloro-5-cyano-N-(2-diethylaminoethyl)- 2-ethoxybenzamide, 4-chloro- 5-cyano-N- (2-diethylaminoethyl)-2-n-propoxybenzamide, 5-chloro-4-cyano-N-(2-diethylaminoethyl)- 2-methoxybenzamide, 4-chloro- 5-cyano-N- (2-diisopropylaminoethyl)-2-methoxybenzamide, 4-chloro- 5-cyano-N-(2-diethylaminoethyl)- 2-isopropoxybenzamide, 4-chloro- 5-cyano- 2-cyclopentyloxy-N- (2-diethylaminoethyl)-benzamide, 4-chloro-5-cyano-2-cyclopropylmethoxy-N- (2-diethylaminoethyl)-benzamide, 2-allyloxy- 4-chloro-5-cyano-N- (2-diethylaminoethyl)-benzamide or a salt thereof is manufactured.

14. A process according to any one of claims 1 to 3, characterised in that a compound of the formula I is manufactured in the form of a pharmaceutically acceptable acid addition salt.

15. A process for the manufacture of pharmaceutical preparations, characterised in that a compound according to any one of claims 1 and 4 to 14, optionally with the admixture of customary adjuncts and carriers, is processed to form pharmaceutical preparations.